(19) Europäisches Patentamt
European Patent Office
Office européen des brevets

(11) **EP 1 240 524 B1**

(12) **EUROPEAN PATENT SPECIFICATION**

(45) Date of publication and mention
of the grant of the patent:
**17.11.2010 Bulletin 2010/46**

(21) Application number: **00984363.2**

(22) Date of filing: **14.12.2000**

(51) Int Cl.:
*G01N 33/68* (2006.01)    *C12Q 1/00* (2006.01)
*C12Q 1/40* (2006.01)    *C12Q 1/37* (2006.01)
*C12Q 1/34* (2006.01)

(86) International application number:
**PCT/US2000/033878**

(87) International publication number:
**WO 2001/047956 (05.07.2001 Gazette 2001/27)**

(54) **CELLULASE FROM T. REESEI WITH IMPROVED THERMOSTABILITY**

CELLULASE VON T. REESEI MIT VERBESSERTER THERMOSTABILITÄT

CELLULASE DE T. REESEI AYANT UNE MEILLEURE THERMOSTABILITE

(84) Designated Contracting States:
**AT BE CH CY DE DK ES FI FR GB GR IE IT LI LU MC NL PT SE TR**

(30) Priority: **23.12.1999 US 470832**

(43) Date of publication of application:
**18.09.2002 Bulletin 2002/38**

(60) Divisional application:
**09014733.1 / 2 184 350**

(73) Proprietor: **GENENCOR INTERNATIONAL, INC.**
**Palo Alto, California 94304 (US)**

(72) Inventors:
• **DAY, Anthony, G.**
**San Francisco, CA 94127 (US)**
• **MITCHINSON, Colin**
**Half Moon Bay, CA 94019 (US)**
• **SHAW, Andrew**
**San Farncisco, CA 94109 (US)**

(74) Representative: **Kiddle, Simon John et al**
**Mewburn Ellis LLP**
**33 Gutter Lane**
**London**
**EC2V 8AS (GB)**

(56) References cited:
WO-A-94/18314    WO-A-95/24471
WO-A-96/23874    WO-A-98/26078
WO-A-99/29876    WO-A2-99/31255

• HOLM L ET AL: "RANDOM MUTAGENESIS USED TO PROBE THE STRUCTURE AND FUNCTION OF BACILLUS-STEAROTHERMOPHILUS ALPHA AMYLASE" PROTEIN ENGINEERING, vol. 3, no. 3, 1990, pages 181-192, XP000087332 ISSN: 0269-2139
• SUZUKI Y ET AL: "AMINO ACID RESIDUES STABILIZING A BACILLUS ALPHA AMYLASE AGAINST IRREVERSIBLE THERMOINACTIVATION" JOURNAL OF BIOLOGICAL CHEMISTRY, vol. 264, no. 32, 1989, pages 18933-18938, XP000872071 ISSN: 0021-9258
• TOMAZIC S J ET AL: "WHY IS ONE BACILLUS ALPHA AMYLASE MORE RESISTANT AGAINST IRREVERSIBLE THERMOINACTIVATION THAN ANOTHER?" JOURNAL OF BIOLOGICAL CHEMISTRY, vol. 263, no. 7, 1988, pages 3092-3096, XP001015592 ISSN: 0021-9258
• NIELSEN JENS ERIK ET AL: "Protein engineering of bacterial alpha-amylases." BIOCHIMICA ET BIOPHYSICA ACTA, vol. 1543, no. 2, 2000, pages 253-274, XP000984337 ISSN: 0006-3002
• SANDGREN ET AL: 'Structural and biochemical studies of GH family 12 cellulases: improved thermal stability, and ligand complexes' PROGRESS IN BIOPHYSICS AND MOLECULAR BIOLOGY vol. 89, 2005, pages 246 - 291

**Description**

## FIELD OF THE INVENTION

The present invention is directed to improved proteins, and particularly enzymes, having altered stability characteristics through the recruitment of residues from a homolog or related protein. Also disclosed is a powerful method which enables the development of such improved proteins with great accuracy.

## BACKGROUND OF THE INVENTION

Methods of improving functional characteristics of proteins have been practiced extensively for the purpose of increasing the usefulness of the proteins in various applications. Such techniques often involve the use of protein engineering and site-specific mutagenesis to isolate and select specific amino acid residues which appear, based on the specific amino acid properties and/or the structure of the protein, to be especially relevant or are shown to be relevant through experimental evidence. For example, Estell et al., U.S. Patent No. 4,760,025 suggest modification of oxidatively unstable enzymes to confer increased stability by altering residues which are particularly susceptible to oxidation, i.e., methionine, lysine, tryptophan and cysteine, among others. Koths et al., U.S. Patent No. 4,752,585, suggest a method of improving the oxidation stability of a therapeutic protein by making a conservative amino acid substitution for each methionyl residue susceptible to chloramine T oxidation. Barr et al., U.S. Patent No. 4,732,973, suggest substituting the active site methionine from human $\alpha_1$-antitrypsin with an oxidatively stable amino acid.

Additional techniques which have been suggested include homology modeling of proteins against evolutionarily close proteins to provide a basis for protein engineering. Siezen et al., Protein Engineering, vol. 4, no. 7, pp. 719-737 (1991) disclose a strategy for improving the properties of proteases used in industrial processes by incorporating or duplicating features from more stable enzymes into a target. Moreover, these techniques require that a attributes from a more desirable protein be imparted to a less desirable protein. In practice, however, the more desirable protein is the target for which it is desired to develop the improved characteristics. Thus, it is not feasible to align such a protein with a more desirable one. This leaves the researcher to look to non-knowledge based techniques for improvement.

Non-knowledge based mutation strategies become necessary where there is a lack of information about the protein of interest or where knowledge based techniques fail. Typical non-knowledge based techniques comprise well known methods including random mutagenesis, error-prone PCR and PCR template switching techniques as well as more recent developments such as shuffling described in Steamer et al., U.S. Patent No. 5,605,793. Random mutagenesis has been used for many years with various levels of success reported throughout the literature. However, random mutagenesis is a hit or miss strategy due to the immense numbers of potential mutants. For example, a 300 amino acid protein has a potential number of random mutants on the order of $300^{20}$. Preparing, expressing and screening such a great number of mutants, even using present day mass screening techniques, is impossible. Directed evolution techniques such as "gene shuffling" as described by Stemmer or other molecular breeding techniques are limited by the available pool of mutants. However, the pool of mutants must be produced either from nature or using random mutagenesis if knowledge regarding the protein is insufficient. This pool of variants, however, potentially falls to provide an optimally focused pool of diverse organisms necessary for a reasonable degree of success.

There has been a considerable effort in the field of protein engineering to produce more stable enzymes. Nonetheless, given the immense information content present in many important proteins, improved methods for intelligently focusing a pool of mutant molecules toward a specific effect must be developed to fully exploit the range of biological diversity. As described below, the present inventors have developed a novel knowledge based mutagenesis technique which provides for excellent probabilities in terms of producing novel proteins which have improved performance, i.e., functional characteristics. By practicing the present technique, it is possible for a researcher to develop a focused pool of mutants from which the selection of a desirable "winner" protein is facilitated.

## SUMMARY OF THE IWENTION

When faced with the problem of improving a protein's performance in regard to a specific functional property, researchers are often confronted with significant hurdles in terms of determining appropriate mutations to make in a protein. The lack of crystal structures for many proteins and the uncertainty involved with low-homology alignments with respect to a comparative protein for which the crystal structure is known are significant impediments to knowledge based mutation strategies. Moreover, random mutagenesis techniques or other non-knowledge based mutation strategies such as molecular breeding are often hit or miss propositions requiring the screening of massive numbers of mutants with no assurance that optimal mutagenesis is even occurring. However, often the researcher seeking to improve a protein in a certain characteristic has at hand a family of related primary structures, most of which are inferior in performance to the protein which is sought to be improved. This naturally flows from the fact that the best protein is often selected as the primary target. However, this "bast protein" often has significant flaws, the improvement of which would have great commercial or Industrial benefit. So the researcher is left with the problem of determining how to modify the protein in a manner which will not detract from the desirable properties of the target protein, but will improve other properties. In this context, the scientists herein determined that by using alignments with less desirable proteins, i.e., less stable or less active enzymes, it is possible to deduce amino acid modifications which will have a great probability of success in terms of being accepted by the "best protein" and not unduly disrupting the good properties thereof. In fact, as nature has often already determined that the selected modification will work in the process for which it is intended, the probability of success is greatly enhanced.

Described herein is a method for obtaining proteins which have improved functional characteristics, including, for example increased alkaline stability, pH stability, thermal stability, increased oxidative stability, increased catalytic activity and improved substrate or target binding.

It is an object of the present invention to provide for a protein, for example an enzyme, having increased alkaline stability, pH stability, thermal stability, increased oxidative stability, increased catalytic activity and improved substrate or target binding.

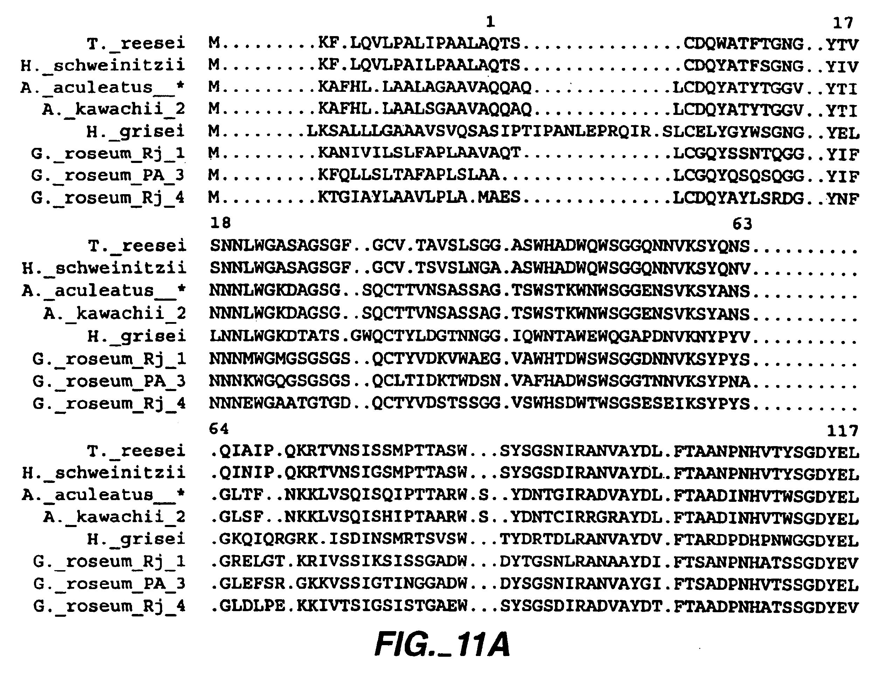

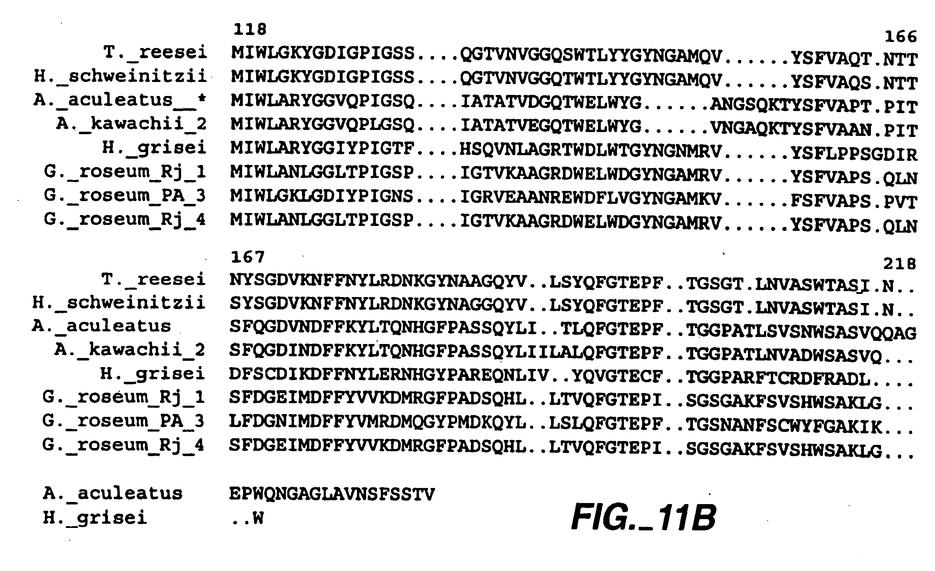

Accordingly in one aspect the invention provides an improved cellulase comprising an amino acid sequence which has been modified from a precursor amino acid sequence having the sequence of *T. reesei* EGIII cellulase shown in Figure 11, said modification comprising a substitution P201C, said amino acid numbering being as in Figure 11; wherein the improved cellulase has increased thermostability compared to a cellulase having the precursor amino acid sequence.

The invention further provides a DNA sequence encoding the cellulase, together with an expression vector comprising the DNA sequence and a host cell comprising the DNA sequence or the expression vector.

Also provided is a method of producing an improved cellulase according to the invention, the method comprising:

(a) modifying a DNA sequence encoding the precursor amino acid sequence to produce a modified DNA sequence according to the invention encoding the improved cellulase;
(b) expressing the modified DNA sequence in a host cell; and
(c) purifying the expressed cellulase protein.

Further provided is use of the improved cellulase in biomass reduction, cleaning products or textile treatment compositions.

WO 95/24471 is concerned with Family 7 cellulases and variants of the cellulases comprising a core and optionally a C-terminal link consisting of 10 amino acids at the most.

WO 99/31255 is concerned with EGIII and EGIII-like cellulases comprising an amino acid string selected from the group consisting of one or more of:

(a) Asn-Asn-(Leu/Phe/Lys/Ile)-Trp-Gly
(b) Glu-(Leu/Phe/Ile)-Met-Ile-Trp
(c) Gly-Thr-Glu-Pro-Phe-Thr
(d) (Ser/Tyr/Cys/Trp/Thr/Asn/Lys/Arg)-(Val/Pro)-(Lys/Ala)-(Ser/Ala)-(Thr/Phe); and
(e) Lys-Asn-Phe-Phe-Asn-Tyr.

Also described herein is a method of improving a desired property of a first protein comprising the steps of: (a) determining the primary amino acid sequence of a first protein and a second homologous protein and aligning said primary amino acid sequences to produce a primary amino acid sequence alignment, said second homologous protein having less desirable characteristics in terms of said desired property than said first protein; (b) identifying residues which differ at corresponding positions between said first protein and said second homologous protein in said primary amino acid sequence alignment; (c) preparing mutant proteins wherein a substitution, deletion or addition is made in said first protein which corresponds to at least one of said residues which differ selected in step (b); (d) screening said mutant proteins for improved performance with respect to said functional property; (e) selecting and determining the identity of said mutant proteins which have such improved performance, wherein said improved mutant protein differs in at least one residue from both said first protein and said second homologous protein and said improved first protein has improved function in terms of said desired property. The alignment of the first protein may be made with more than one second homologous proteins and the substitutions selected in step (c) may be chosen from those residues which exist in a significant portion or all of the second homologous proteins at the specific position selected for mutation. The improved protein may have improved stability properties, either alkaline, pH, thermal or oxidative, compared to a protein corresponding to the precursor amino acid sequence. Also, the protein may be an enzyme, for example an α-amylase, lipase, cellulase or protease.

Also described is, a method for the production of a target protein having an improvement in a desired property compared to a first protein provided comprising: (a) aligning the primary amino acid sequences of at least two comparative proteins, one of which comparative proteins has less desirable performance than the other in terms of said desired property; (b) identifying positions at which the residues differ between said two comparative enzymes and determining the residues which are present at such identified positions In the comparative protein which has less desirable performance in terms of said desired property; (c) selecting one or more of the residues determined in step (b); (d) modifying said first protein at a position corresponding to the position in said comparative protein at which residues are selected in step (c) and substituting, deleting or adding said selected residue in said first protein; wherein said improved target protein differs in at least one residue from both said first protein and said comparative proteins and said target protein has improved function in terms of said desired property.

The comparison may be made with more than one second homologous protein and a residue existing only in all or several of the less stable second homologous proteins may be recruited into the first protein to make the improved protein.

Thus described herein is an improved protein obtained according to a method described herein wherein said protein comprises a modification according to a method described.

An advantage of the methods described is that it is possible to produce proteins having more desirable properties through a simple technique of analyzing the aligned sequences of two homologous proteins which differ in terms of said properties.

Another advantage is that it is possible to significantly reduce the pool of potential mutants to a more easily screened number of mutants as opposed to non-knowledge based methods of mutation.

Yet another advantage is that no information is necessary regarding the tertiary structure of the protein to be improved, merely a sequence alignment with homologous protein.

Yet another advantage is that it is possible to further improve a superior protein based on information obtained from less desirable proteins, thus increasing the pool of available information for developing such improved proteins.

## BRIEF DESCRIPTION OF THE DRAWINGS

Figure 1 Illustrates mutagenic oligonucleotides useful during directed mutagenesis of Asn188 from *Bacillus licheniformis* α-amylase. In this and following figures illustrating oligonucleotide constructs, bold letters indicate base changes introduced by the oligonucleotide and underlining indicates restriction endonuclease sites introduced by the oligonucleotide. -

Figure 2 illustrates PCR primers used for PCR processing of mutagenic oligonucleotide templates.

Figure 3 illustrates the DNA sequence of the gene for α-amylase from *Bacillus licheniformis* (NCIB 8061) (SEQ ID NO:33) and deduced amino acid sequence of the translation product (SEQ ID NO:41) as described by Gray et al., J. Bacteriology, vol. 166, pp. 635-643 (1986).

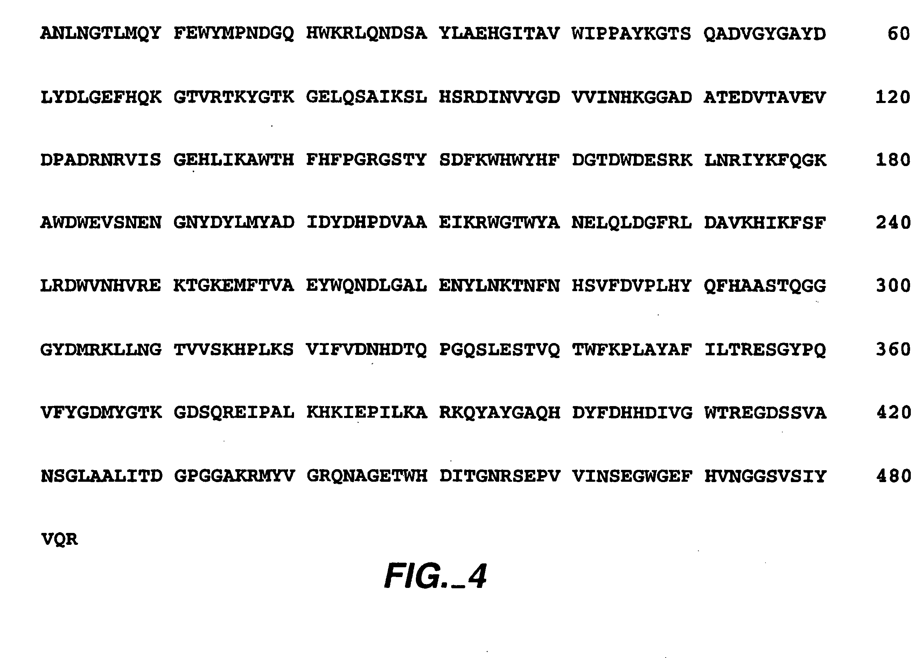

Figure 4 illustrates the amino acid sequence (SEQ ID NO:34) of the mature α-amylase enzyme from *Bacillus licheniformis*.

Figure 5 illustrates an alignment of the primary structures of three *Bacillus* α-amylases. The *Bacillus licheniformis* α-amylase (Am-Lich) (SEQ ID NO:35) is described by Gray et al., J. Bacteriology, vol. 166, pp. 635-643 (1986); the *Bacillus amyloliquefaciens* α-amylase (Am-Amylo) (SEQ ID NO:36) is described by Takkinen et al., J. Biol. Chem., vol. 258, pp. 1007-1013 (1983); and the *Bacillus stearothermophilus* α-amylase (Am-Stearo) (SEQ ID NO:37) is described by Ihara et al., J. Biochem., vol. 98, pp. 95-103 (1985).

Figure 6 illustrates plasmid pHP13 wherein Cm$^R$ refers to chloramphenicol resistance, Em$^R$ refers to erythromycin resistance and Rep pTA1060 refers to the origin of replication from plasmid pTA1060.

Figure 7 illustrates the pBLapr plasmid wherein BL AA refers to *Bacillus licheniformis* α-amylase gene; *aprE* refers to the promoter and signal peptide encoding region of the *aprE* gene; AmpR refers to the ampicillin resistant gene from pBR322; and CAT refers to the chloramphenicol resistance gene from pC194.

Figure 8 illustrates the pHP.BL plasmid carrying the gene for *Bacillus licheniformis* α-amylase.

Figure 9 illustrates a schematic of the PCR method used to produce the mutant oligonucleotides corresponding to α-amylase derived from *Bacillus licheniformis.*

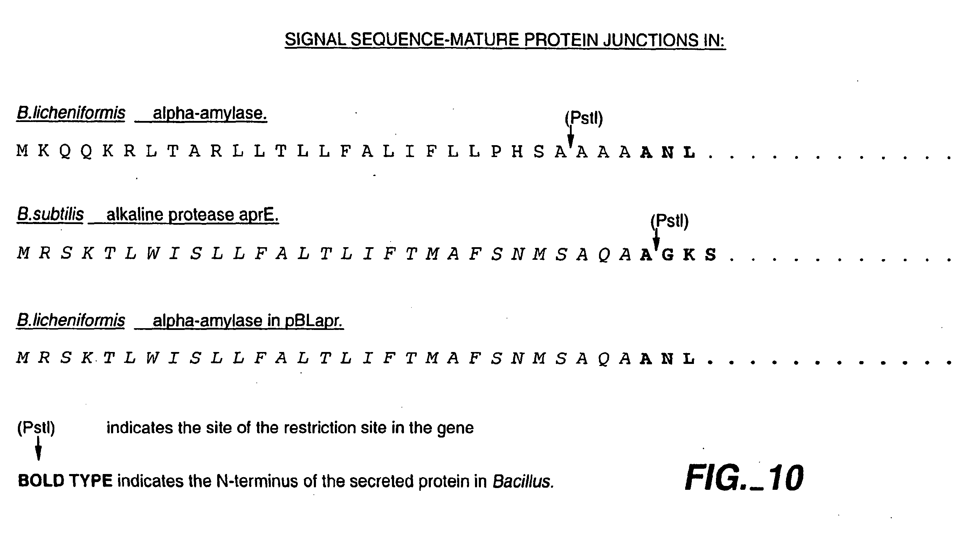

Figure 10 illustrates the signal sequence-mature protein junctions in α-amylase derived from *Bacillus licheniformis* (SEQ ID NO:38), *Bacillus subtilis aprE* (SEQ ID NO: 39) and *Bacillus licheniformis* in pBLapr (SEQ ID NO:40).

Figure 11 illustrates a sequence alignment of 8 homologous endoglucanase enzymes corresponding to certain cellulases obtained from *Trichoerma reesei (EGIII)*(SEQ. ID NO: 41), *Hypocrea schweinitzii* (SEQ. ID. NO: 42), *Aspergillus aculeatus* (SEQ. ID. NO: 43), *Aspergillus kawachii* (SEQ. ID. NO: 44), *Humicola grisea* (SEQ ID NO: 45), *Gliocladium roseum (1)* (SEQ. ID. NO:46), *Gliocladium roesum (2)* (SEQ. ID. NO: 47), *Gliocladium roseum (3)* (SEQ. ID. NO:48). Numbering provided is based on the numbering in EGIII.

## DETAILED DESCRIPTION

"Expression vector" means a DNA construct comprising a DNA sequence which is operably linked to a suitable control sequence capable of effecting the expression of said DNA In a suitable host. Such control sequences may include a promoter to effect transcription, an optional operator sequence to control such transcription, a sequence encoding suitable mRNA ribosome-binding sites, and sequences which control termination of transcription and translation. Where expression is desired in a *Bacillus* host, a preferred promoter is the *Bacillus subtilis aprE* promoter. The vector may be a plasmid, a phage particle, or simply a potential genomic insert. Once transformed into a suitable host, the vector may replicate and function independently of the host genome, or may, in some instances, integrate into the genome itself. While plasmid and vector are sometimes used interchangeably as the plasmid is the most commonly used form of vector at present, the invention is intended to include such other forms of expression vectors which serve equivalent functions and which are, or become, known in the art.

"Host strain" or "host cell" means a suitable host for an expression vector comprising DNA encoding a protein according to the present invention. Host cells useful in the present invention are generally procaryotic or eucaryotic hosts, including any transformable microorganism in which the expression of a given protein according to the present invention can be achieved. The host cells are not the human body at the various stages of its formation and development. One of skill in the art will be familiar with the appropriate expression and secretion machinery, including the appropriate host cell, for use with a specific protein. For example, host strains of the same species or genus from which the particular protein is derived are suitable, for example with $\alpha$-amylase derived from *Bacillus,* a suitable host cell would be a *Bacillus* strain. In this case, an $\alpha$-amylase negative *Bacillus* strain (genes deleted) and/or an $\alpha$-amylase and protease deleted *Bacillus* strain ($\Delta amyE$, $\Delta apr$, $\Delta npr$) is preferably used. Host cells are transformed or transfected with vectors constructed using recombinant DNA techniques. Such transformed host cells are capable of either replicating vectors encoding the protein and its variants (mutants) or expressing the desired protein.

"Recombinant protein" means a protein in which the DNA sequence encoding the naturally occurring or precursor protein is modified to produce a mutant DNA sequence which encodes the substitution, insertion or deletion of one or more amino acids in the protein sequence compared to a naturally occurring or precursor protein. As used herein, the term precursor protein (or enzyme) refers not to an immediate precursor in terms of a chemical reaction, but instead to a parent protein from which the modification is modeled. Accordingly, while it is the precursor which defines the modification, the actual alteration or modification will have its basis in an alteration within the DNA which encodes the precursor, which DNA is then transformed, expressed and the protein product secreted which incorporates the modification.

The improved protein according to the present invention comprises an amino acid sequence which is derived from the amino acid sequence of a precursor protein (also referred to herein as the "first protain"), the specific modifications in the precursor protein being as provided herein. The precursor protein may be a naturally occurring protein or a recombinant protein. The amino add sequence of the improved protein is derived from the precursor protein's amino acid sequence by the substitution, deletion or insertion of one or more amino adds of the precursor amino acid sequence. Such modification is generally of the precursor DNA sequence which encodes the amino add sequence of the precursor proteins rather than manipulation of the precursor protein *per se*. Suitable methods for such manipulation of the precursor DNA sequence include methods disclosed herein and in commonly owned U.S. Patent Nos. 4,760,025 and 5,185,258, Incorporated herein by reference.

"Desired properties" means any property for which it is desired to improve a protein. Such desired properties may comprise functional properties such as thermal, pH, alkaline, oxidative or chemical stability, catalytic activity, substrate binding, receptor binding, allergenicity, antimicrobial activity or any other desirable feature associated with a protein.

"Primary amino acid sequence alignment" means an alignment of the primary sequence of at least two proteins in terms to produce a significant degree of sequence identity. Different alignment algorithms exist in the form of commercially available software which are suitable and are routine in use to the person of ordinary skill in the art. These programs provide for the input of different algorithm parameters which may effect the results obtained. However, the present invention does not require a preference for any specific sequence alignment algorithm as the choice is generally one of optimizing the parameters. In general, alignments are considered approximate, and thus using different methods of optimizing the alignment should advantageously provide slightly different pools of mutants to select from. Nonetheless, because an advantage of the present invention is to produce a focused pool of mutant proteins from which to select improved proteins, the present invention should be equally applicable regardless of the program or parameters used to determine the alignment.

According to one method described herein, a protein is modified as follows to improve its performance in terms of a desired property. The primary amino acid sequence of the protein to be modified, called the "first protein", Is aligned with the primary amino acid sequence of a comparative "second homologous protein" which has less desirable performance in terms of the desired property. From the alignment, positions at which residues differ are determined and the residues present in the second homologous protein sequence at those positions are identified. At least one of the selected residues from the previous step is then incorporated into the amino acid sequence of the first protein. As determined by the inventors, the modified protein produced in this manner has a significant probability of having improved performance in terms of the desired property. Accordingly, a pool of mutant proteins produced in this manner having various combinations of identified residues substituted, deleted or added will, in all likelihood, comprise a pool of improved proteins which can then be selected among for the "winner".

In a variation, it is possible to produce alignments of more than two primary amino acid sequences. In this method, the first protein is aligned with more than one second homologous protein primary amino acid sequence, each second homologous protein primary amino acid sequence corresponding to a different protein which has less beneficial activity than that of the first protein. From this alignment residues are selected which exist in one or more of the second homologous proteins at positions in which there is a difference between the first protein primary amino acid sequence and at least two of the second homologous protein primary amino acid sequences. In one alternative which expands the pool of mutants available, each of the variations between the first protein a second homologous protein can be used as the basis for producing a mutant. In another preferred alternative which limits the number of mutants available to those most likely to successfully impart improved functional characteristics on the mutant proteins selected, only those differences in which each of the second homologous protein sequences comprise an identical residue are selected.

In one variation of the above method, the alignment is prepared with two or more homologous proteins which are not directly compared to the first protein. For example, two comparative homologous proteins, each of which are capable of being aligned with the first protein, are aligned to determine where residues differ. The resulting alignment is then analyzed to determine the residues which exist in the comparative protein or proteins having less desirable performance in terms of the desired property, and that residue is then substituted, deleted or added into the first protein at a corresponding position. In this method, it is not necessary that the comparative proteins be better or worse than the first protein, only that the selected residue for modification in the target protein is derived from the homologous comparative protein which has less desirable performance in terms of the desired property. In addition, It is possible to align more than two second homologous protein primary amino acid sequences and to select from among them based on the performance of the various second homologous proteins in terms of the desired property.

Applicants have found that a protein having an improved functional property may be obtained by preparing an alignment of two or more homologous or related proteins and referencing the residues which exist at various locations in the proteins having less desirable performance in terms of a desired property. For example, by comparing a first protein against a second homologous protein that has less desirable characteristics in terms of the functional property, identifying residues which differ, preparing a pool of mutants based on said differing mutations, and selecting the mutants which are improved in terms of the desired property in comparison with the first protein, advantageous improvements in desired properties may be obtained in a significant percentage of such modifications. Thus, one excellent use of the present methods is to produce a relatively small and manageable pool of mutants (in comparison with the pool of random mutants) from which it Is possible to select a protein having significantly improved functional properties when compared to the first protein. Moreover, this result is possible without having intimate structure-function information.

It Is not necessary that the aligned proteins have comparable activity and function. In a preferred example, however, the proteins have comparable activity, i.e., similar biological activity, function, catalytic activity or such other criteria as are commoniv used to classify a specific protein. However, as the present methods are based on determining positions at which nature has determined a residue can exist in the context of a protein without significantly effecting its value to a host organism, it is not necessary that all features of the compared proteins be identical, merely that they have some homology. "substantially homologous" means that the proteins have a significant level of conserved, i.e., identical, amino acids such that their sequences can be meaningfully aligned and major structural, functional or catalytic sites defined. Preferably, the first protein and the second homologous proteins have a sequence identity of at least 30%, preferably 50% sequence identity, more preferably 70% sequence identity and most preferably 85% sequence identity.

In one example , the protein comprises an enzyme. The enzyme may comprise any enzyme In the five major enzyme classifications of hydrolase, oxidoreductase, transferase, lyase or ligase. Specific examples of enzymes which may benefit from the present methods include amylase, lipase, cellulase, protease, hemicellulase, glucoamylase, esterase, lactase, polygalacturonase, β-galactosidase, ligninase, oxidase, peroxidase, glucose isomerase or any enzyme for which closely related and loss stable homologs exist.

α-Amylase will be illustrated below as exemplary of the present methods and compositions. "α-Amylase" as used herein means an enzymatic activity which cleaves or hydrolyzes the α(1-4)glycosidic bond, e.g., that in starch, amylopectin or amylose polymers. α-Amylase includes naturally occurring α-amylases as well as recombinant α-amylases. Preferred α-amylases in the present invention are those derived from *Bacillus sp.,* particularly those from *Bacillus licheniformis, Bacillus amyloliquefectens* or *Bacillus stearothermophllus,* as well as fungal α-amylases such as those derived from *Aspergillus* (i.e., *A. oyzee* and *A. niger*). Accordingly, the α-amylases according to the present methods are derived from a precursor α-amylase. The precursor α-amylase is produced by any source capable of producing α-amylase. Suitable sources of α-amylases are prokaryotic or eukaryotic organisms, including fungi, bacteria, plants or animals. Preferably, the precursor α-amylase is produced by a *Bacillus*; more preferably, by *Bacillus licheniformis, Bacillus amylollquefaciens* or *Bacillus stearothemophilus*; most preferably, the precursor α-amylase is derived from *Bacillus licheniformis.*

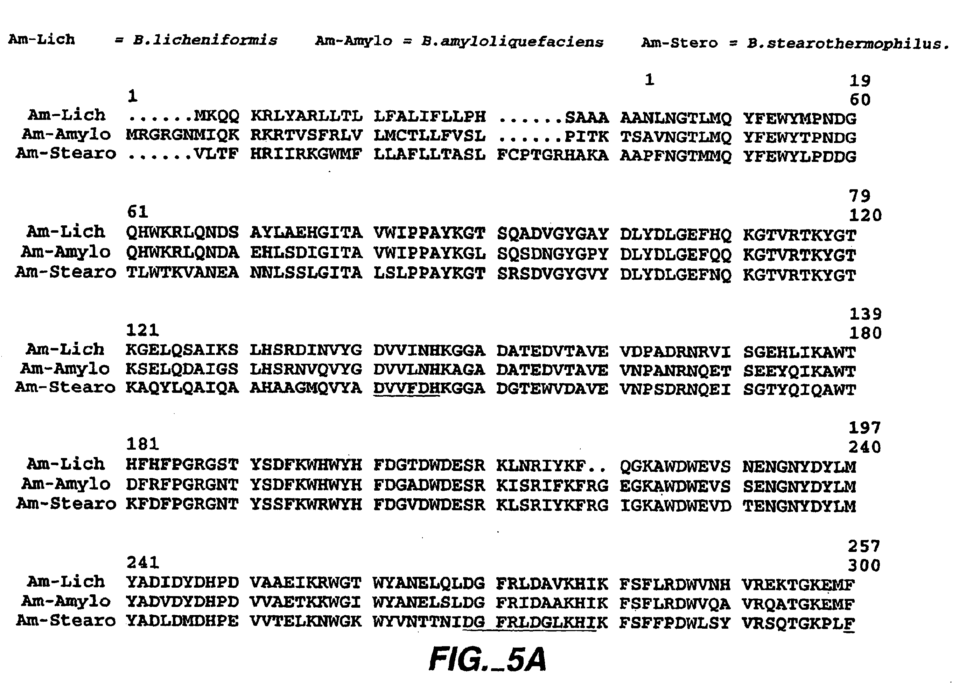

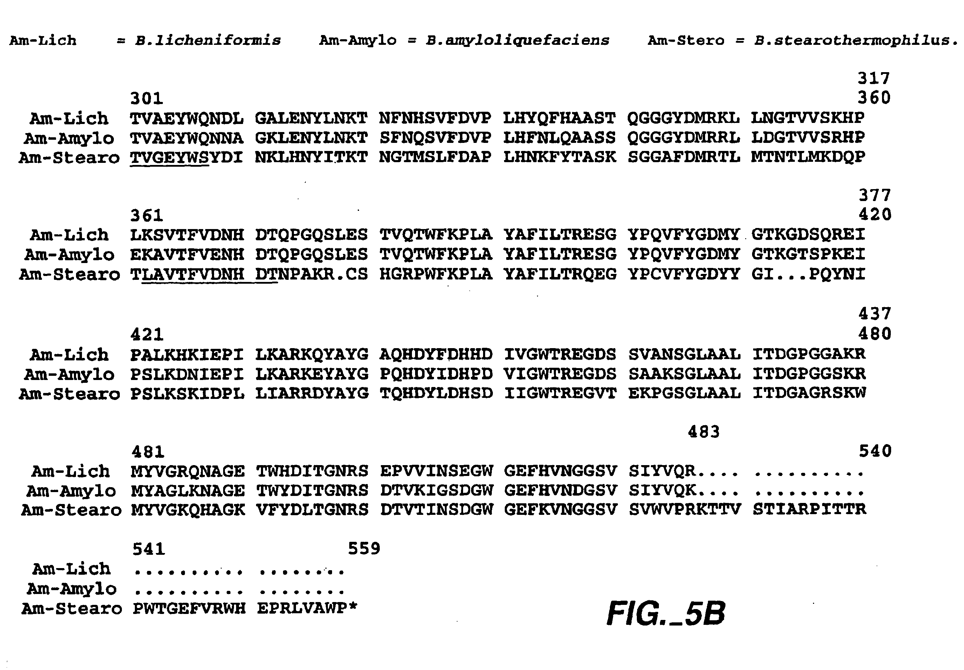

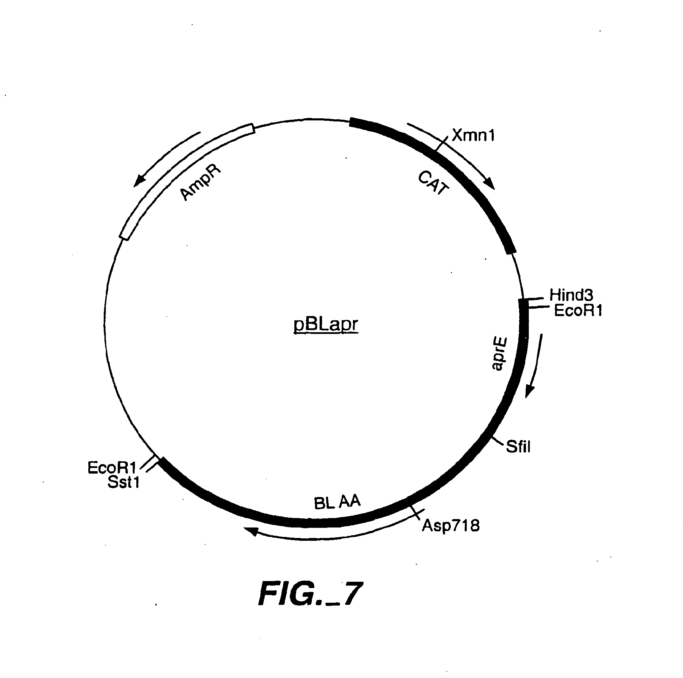

Homologies have been found between almost all endo-amylases sequenced to date, ranging from plants, mammals, and bacteria (Nakajima et al.; Appl. Microbiol. Biotechnol., vol. 23, pp. 355-360 (1986); Rogers, Blochem. Blophys. Res. Commun., vol. 128, pp. 470-476 (1985); Janecek, Eur. J. Biochem., vol. 224, pp. 519-524 (1994)). There are four areas of particularly high homology In certain *Bacillus* amylases, as shown in Figure 5, wherein the underlined sections designate the areas of high homology. Sequence alignments have also been used to map the relationship between *Bacillus* endo-amylases (Feng et al., J. Molec. Evol., vol. 35, pp. 351-360 (1987)). The relative sequence homology between *Bacillus stearothermophilus* and *Bacillus licheniformis* amylase is about 66% and that between *Bacillus licheniformis* and *Bacillus amyloliquefaciens* amylases is about 81%, as determined by Holm at al., Protein Engineering, vol. 3, No. 3, pp. 181-191 (1990). In order to establish homology to primary structure, the amino acid sequence of a precursor α-amylase is directly compared to the *Bacillus lichenlformis* α-amylase primary sequence and particularly to a set of residues known to be invariant to all α-amylases for which sequences are known (see e.g., Figure 7). It Is possible also to determine equivalent residues by tertiary structure analysis of the crystal structures reported for porcine pancreatic α-amylase (Buisson et al., EMBO Journal, vol. 6, pp. 3909-3916 (1987); Qlan et al., Biochemistry, vol. 33, pp. 6284-6294 (1994); Larson et al., J. Mol. Biol., vol. 235, pp. 1560-1584 (1994)); Taka-amylase A from *Aspergillus oryzae* (Matsuura et al., J. Blochem. (Tokyo), vol. 95, pp. 697-702 (1984)); and an acid α-amylase from *A. niger* (Boel et al.. Biochemistry, vol. 29, pp. 6244-6249 (1990)), with the former two structures being similar, and for barley α-amylase (Vallee et al., J. Mol. Biol., vol. 236, pp. 368-371(1994); Kadzlola, J. Mol. Biol., vol. 239, pp. 104-121 (1994)). Although there have been some preliminary studies published (Suzuki et al, J. Blochem., vol. 108, pp. 379-381 (1990); Lee et al., Arch. Biochem. Blophys, vol. 291, pp. 255-257 (1991); Chang et al, J. Mol. Biol., vol. 229, pp. 235-238 (1993); Mizuno et al., J. Mol. Biol., vol. 234, pp. 1282-1283 (1993)), there is only a published structure for *Baclillus licheniformis* α-amylase (Machlus et al., J. Mol. Biol. vol. 246, pp. 545-549 (1995)). However, several researchers have predicted common super-secondary structures between glucanases (MacGregor et al., Biochem. J., vol. 259, pp. 145-152 (1989)) and within α-amylases and other starch-metabollsing enzymes (Jaspersen, J. Prot. Chem. vol. 12, pp. 791-805 (1993); MacGregor, Starke, vol. 45, pp. 232-237 (1993)); and sequence similarities between enzymes with similar super-secondary structures to α-amylases (Janecek, FEBS Letters, vol. 316, pp. 23-26 (1993); Janecek et al., J. Prot. Chem., vol.12, pp. 509-514 (1993)). A structure for the *Bacillus stearothermophilus* enzyme has been modeled on that of Taka-amylase A (Holm et al., Protein Engineering, vol. 3, pp.181-191 (1990)). The four highly conserved regions shown In Figure 7 contain many residues thought to be part of the active-site (Matsuura et al., J. Blochem. (Tokyo), vol. 95, pp. 697-702 (1984); Buisson et al., EMBO Journal, vol. 6, pp. 3909-3916 (1987); Vihinen et al., J. Biochem., vol.107, pp. 267-272 (1990)) including His +105; Arg +229; Asp +231; His +235; Glu +261 and Asp +328 under the *Bacillus lichenlformis* numbering system. The crystal structure of a bacterial amylase hybrid enzyme has been published In PCT Publication No. WO 96/23874.

As described above, the α-amylases from *Bacillus licheniformis, Bacillus stearothermophilus, Bacillus amyloliquefaciens* and *Bacillus subtilis* all bear a significant degree of homology. However, It is known that under the conditions of Industrial starch liquefaction, e.g.. high temperature (excess of 90°C), low pH (pH 4-6) and low calcium, α-amylase derived from *Bacillus licheniformis* provides the most acceptable performance, Nonetheless, even *Bacillus lichenifomris* α-amylase is susceptible to undesirable instability under liquefaction conditions making a more stable alternative desirable. Accordingly, much work has been performed on the *Bacillus licheniformis* derived molecule for the purpose of introducing Into this enzyme properties which are desirable in connection with its use in liquefaction processes. By aligning the sequence of α-amylase from *Bacillus licheniformi* against that of α-amylase from either *Bacillus stearothermophilus* or *Bacillus amylollquefaciens* it is possible to identify residues which differ between the homologs. According to the present methods, then, the positions at which residues differ in α-amylase from *B. stearothermophilus* or *B. amyloliquefaciens* compared to α-amylase from *B. lichenlformis* are selected for substitution In α-amylase from *B. licheniformis.* The substituted residue may be actually an identical residue as that which exists in α-amylase from either *B. stearothermophilus* or *B. amyloliquefaciens.* The substituted residue may correspond to a position in which the same residue exists in both α-amylase from *B. stearothermophilus* and *B. amyloliquefaciens.*

Residues specifically identified herein for replacement in *Bacillus lichanitormis* are those which differ from residues in a corresponding position In *Bacillus amyloliquefaciens* and/or *Bacillus stearothermophilus,* and particularly A33, A52, S85, N98, H133, S148, A209, A269, A379 and A435. Specific preferred replacements for these residues are selected from those present In both *Bacillus amyloliquefaciens* and *Bacillus stearothermophilus* and correspond to A33S, A52S, N96Q, H133Y, S148N, A209V, A269K, A379S and/or A435S all appear to contribute to a stability benefit. It has also been discovered that the A85D mutation, which is recruited only from *Bacillus amyloliquefaciens* also provides a stability benefit. The above residues may be altered in combination with other modiflcations which provide a performance benefit.

The improved proteins according to the present methods exhibit improved performance characteristics which make those proteins particularly useful in various applications for which the proteins are commonly used and for which improved stability is desired. For example, enzymes, including α-amylases, will exhibit improved thermostability, improved pH stability and/or improved oxidative stability. Enhanced thermostability will be useful in extending the shelf life of products which incorporate them and for applications at high temperatures. Enhanced oxidative stability or improved performance is particularly desirable in cleaning products, and for extending the shelf life of the enzyme in the presence of bleach, perborate, percarbonate or peracids used in such cleaning products. An α-amylase as described herein is especially useful in starch processing and particularly in starch liquefaction wherein oxidative and thermal stability are particularly important. Cellulases having improved stability benefits are also disclosed herein and are particularly useful in, for example, biomass reduction, cleaning products and textile treatment compositions.

An additional embodiment of the present invention comprises DNA encoding a protein according to the present invention and expression vectors comprising such DNA. The DNA sequences may be expressed by operably linking them to an expression control sequence in an appropriate expression vector and employing that expression vector to transform an appropriate host according to well known techniques. A wide variety of host/expression vector combinations may be employed in expressing the DNA sequences of this invention. Useful expression vectors, for example, include segments of chromosomal, non-chromosomal and synthetic DNA sequences, such as the various known plasmids and phages useful for this purpose. In addition, any of a wide variety of expression control sequences are generally used in these vectors. For example, with α-amylase derived from *Bacillus,* Applicants have discovered that a preferred expression control sequence for *Bacilus* transformants is the *aprE* signal peptide derived from *Bacillus subtills*.

A wide variety of host cells are also useful in expressing the DNA sequences of this invention. These hosts may include well known eukaryotic and prokaryotic hosts, such as strains of *E. coil, Pseudomonas, Bacillus, Streptomyces,* various fungi, yeast and animal cells. Preferably, the host expresses the protein of the present invention extracellularly to facilitate purification and downstream processing. Expression and purification of the improved protein of the invention may be effected through art-recognized means for carrying out such processes.

The following is presented by way of example and is not to be construed as a limitation to the scope of the claims. Abbreviations used herein, particularly three letter or one letter notations for amino acids are described in Dale, J.W., Molecular Genetics of Bacteria, John Wiley & Sons, (1989) Appendix B.

## EXAMPLES

Reference EXAMPLE 1

Construciton Of Plasmld pHP.BL

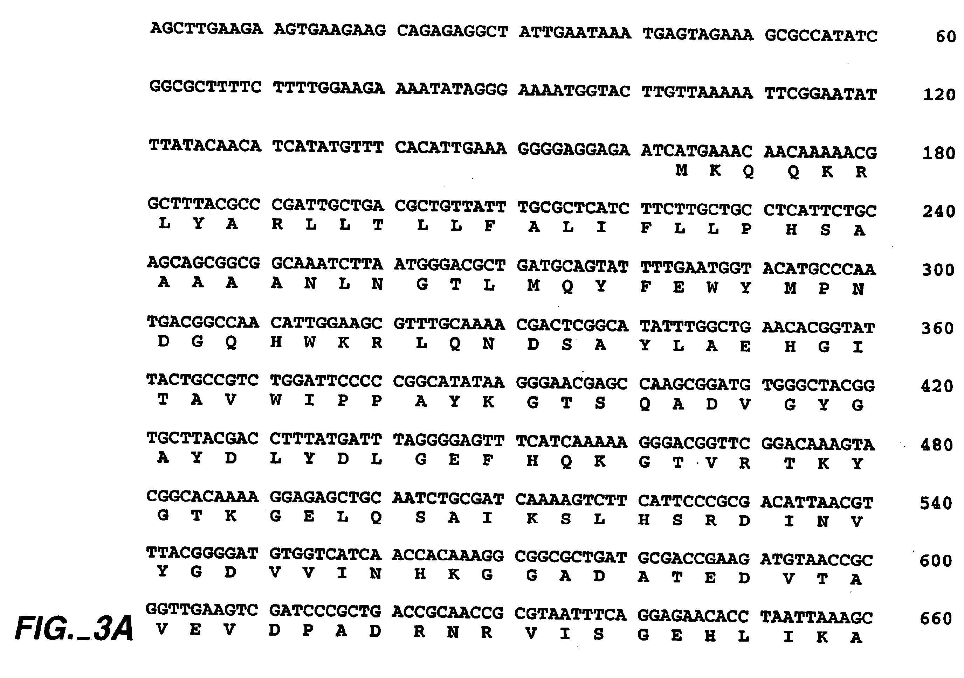

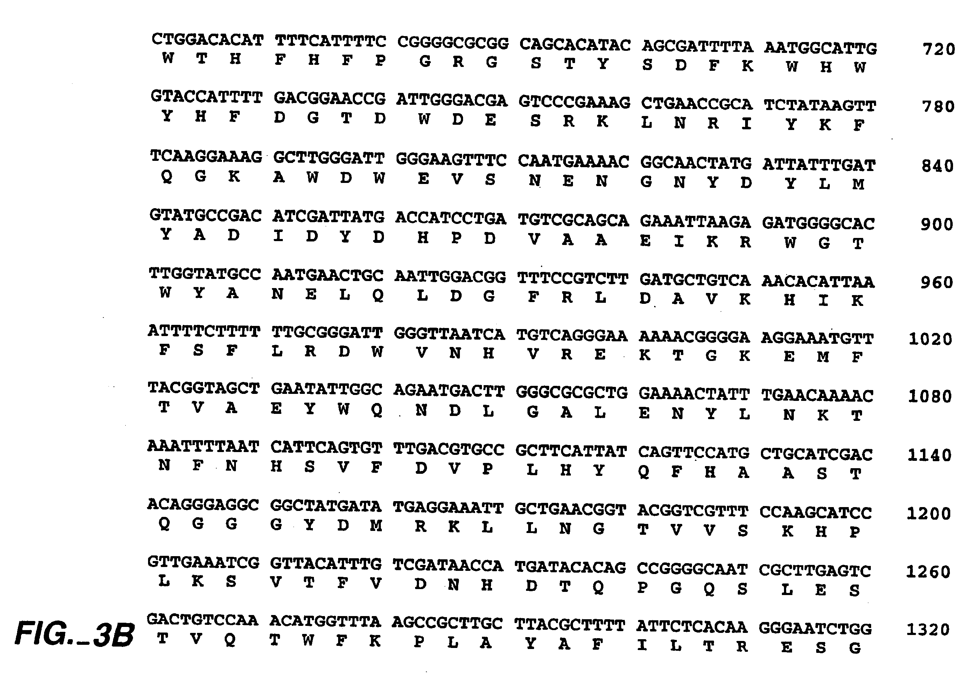

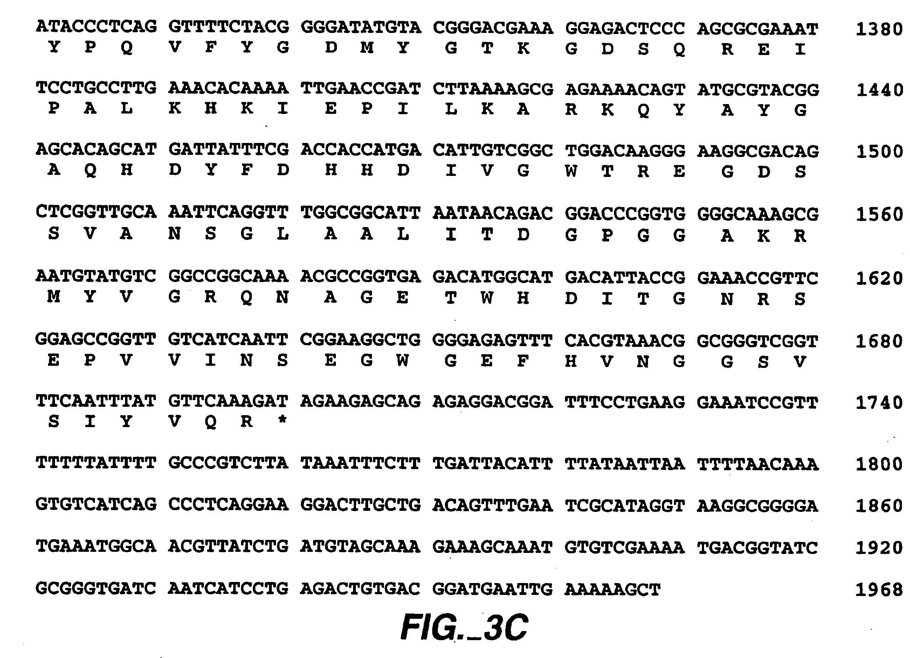

The α-amylase gene shown in Figure 3 was cloned from *Bacillus lichenifomris* NCiB8061 (Gray et al., J. Bacteriology, vol. 166, pp. 635-643 (1986)). The 1.72kb.Psti-Ssti fragment, encoding the last three residues of the signal sequence, the entire mature protein and the terminator region, was subcloned Into M13mp18. A synthetic terminator was added between the BclI and SstI sites using a synthetic oligonucleotide cassette of the form:

```
BclI                                                                                    SstI
5'-GATCAAAACATAAAAAACCGGCCTTGGCCCCGCCGGTTTTTTATTATTTTTGAGCT-3'   (SEQ ID NO:1)
3' TTTTGTATTTTTTTGGCCGGAACCGGGGCGGCCAAAAAATAATAAAAAC 5'          (SEQ ID NO:2)
```

designed to contain the *Bacillus amyloliquefaclens* subtilisin transcriptional terminator (Wells et al., Nucleic Acid Research, vol. 11, pp. 7911-7925 (1983)).

The pBLapr plasmid was constructed carrying the gene for the B*acillus licheniformis* α-amylase. As illustrated in Figure 7, pBLapr comprises a 6.1kb plasmid including the ampiclilin resistance gene from pBR322 and the chloramphenicol resistance gene from pC194, the aprE promoter and the gene encoding for the *Bacillus lichenifonnis* α-amylase (BL AA"). The aprE promoter was constructed from a 660bp Hindill-Psti fragment encoding for the promoter and signal sequence of the *Bacillus subtilis* alkaline protease. The Pstl site was removed, and an Sfll site added dose to the *aprE/ BL* AA junction. The BL AA gene comprises the 1720 bp Pstl-Sstl fragment described above. In the work described herein, pBLapr was constructed with an Sfil site adjacent to the 5' end of the start of the coding sequence for the mature amylase gene. Specifically, the 5' end of the pBLapr construction was subcloned on an EcoRI-SstII fragment from pBLapr into M13BM20 (Boehringer Mannheim) to obtain a coding-strand template for the mutagenic oligonucleotide below:
5'- CCC ATT AAG ATT GGC CGC CTG GGC CGA CAT GTT GCT GG - 3' (SEQ ID NO:3) This primer introduced an Sfil site (indicated by underlining) which allowed correct forms to be screened for by the presence of this unique restriction site. Subcloning the EcoRi-Sstil fragment back into the pBLapr vector gave a version of the plasmid containing an Sfil site.

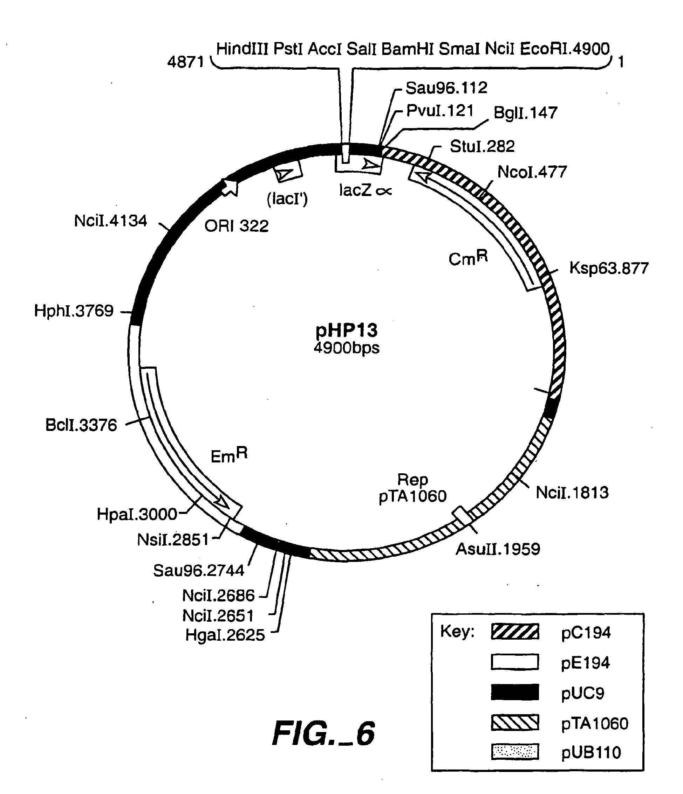

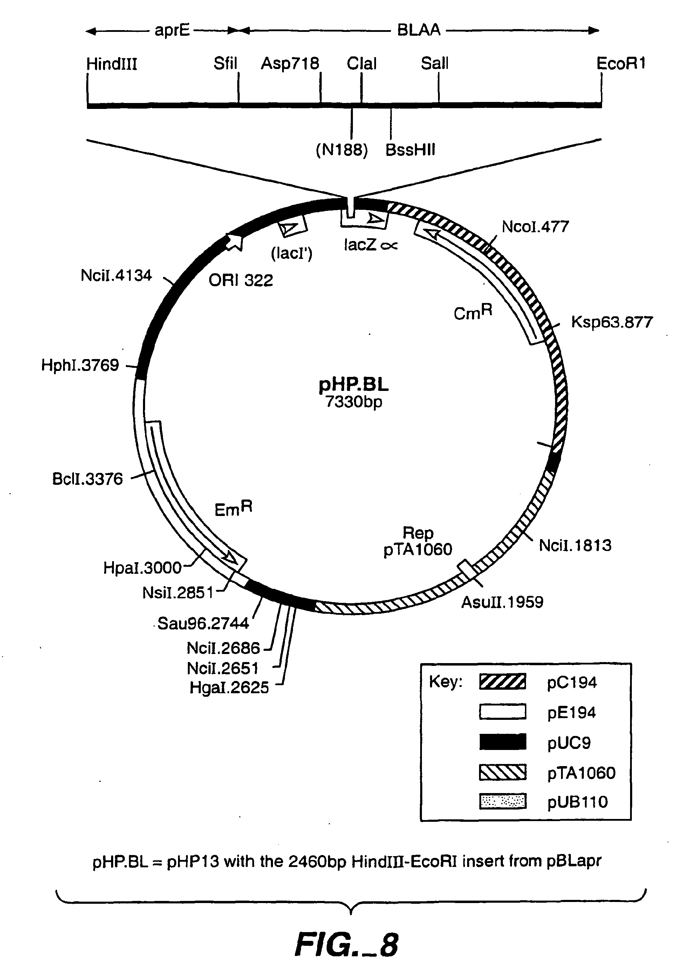

Plasmid pHP13 (Haima et al., Mol. Gen. Genet., vol. 209, pp. 335-342 (1987)) (Figure 6) was digested with restriction enzymes EcoRI and HindIII and the resulting vector purified on a polyacrymide gel and then eluted. Plasmid pBLapr was digested with HindIII, Asp718 and in a separate incubation with Asp718, EcoRI and gel purified. Two bands, HindIII-Asp718 (1203 bp) and Asp718-EcoRI (1253 bp)were gel purified, eluted from the gel and ligated into the vector by a 3-way ligation, to give plasmid pHP.BL, the plasmid used in expression of the α-amylase (Figure 8).

Reference EXAMPLE 2

Construction Of Plasmid Encoding α-Amylase Comprising Substitutions For Asparagine 188

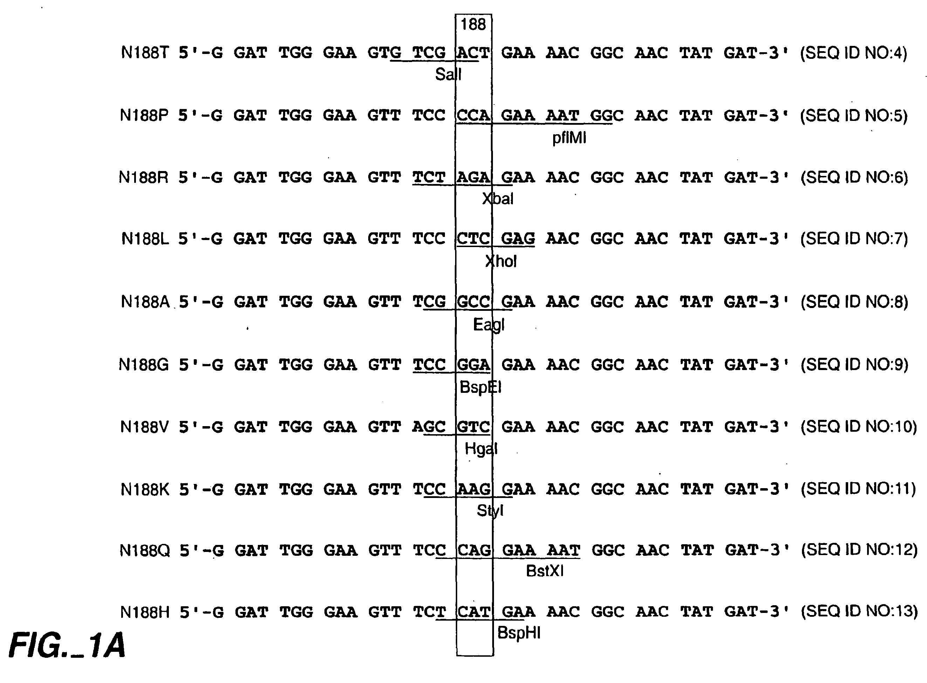

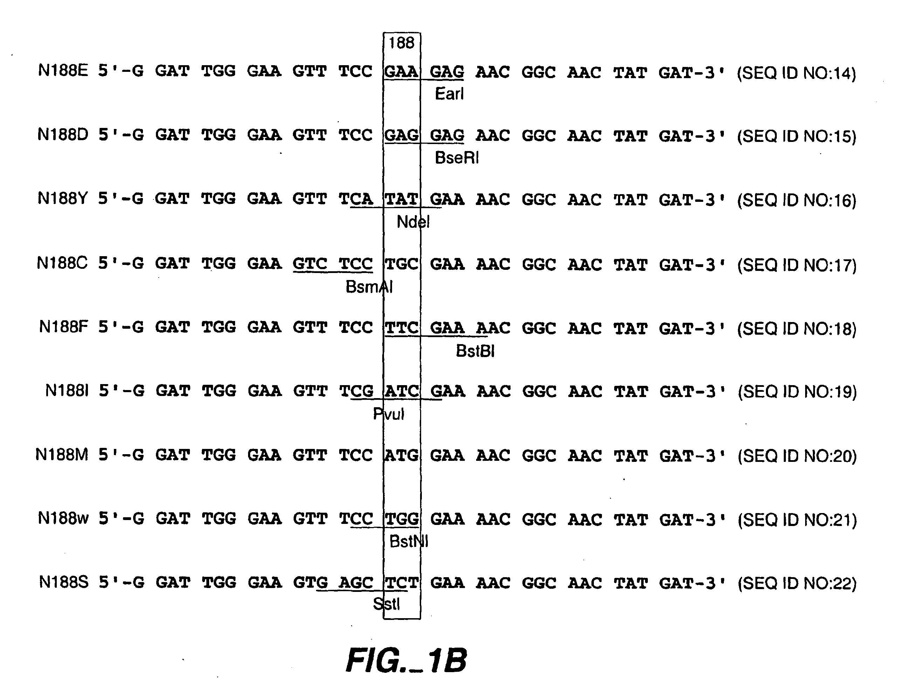

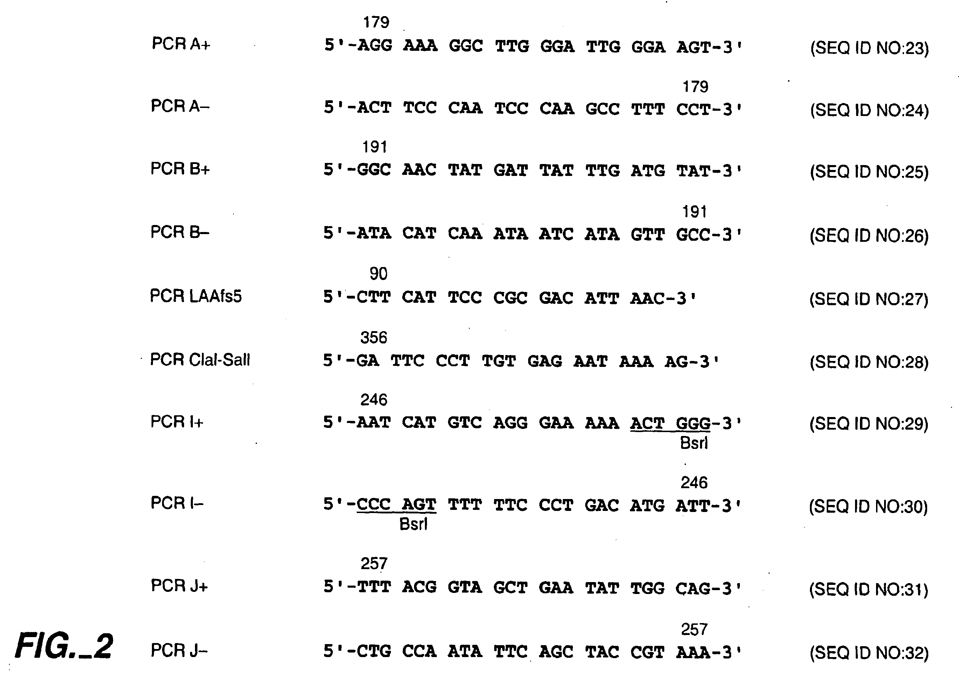

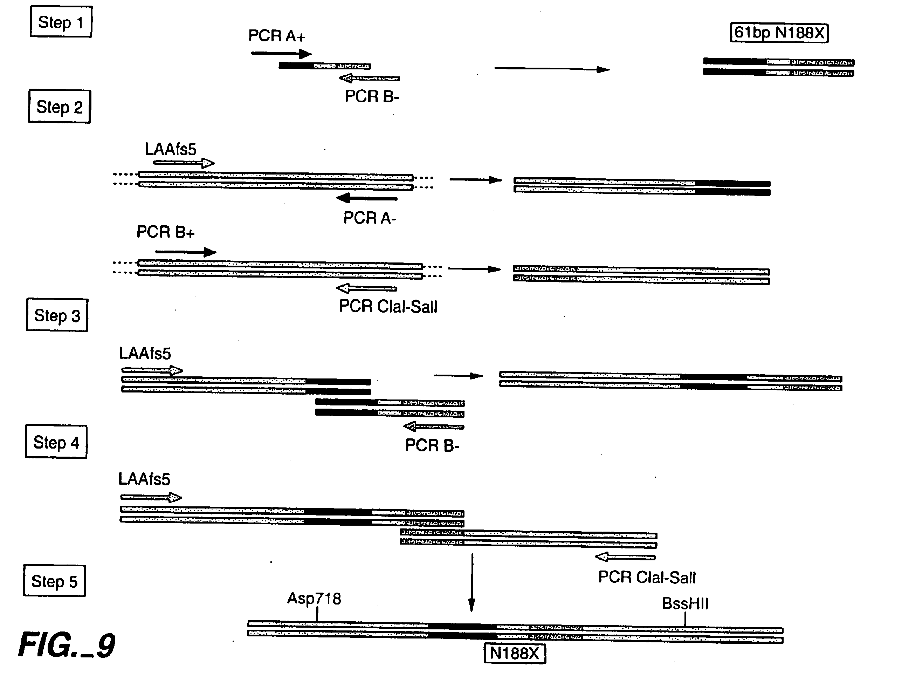

A series of mutagenic primers encoding for substitutions of Asn188 ("N188") with each of the naturally occurring amino acids were synthesized and are shown in Figure 1 (SEQ ID NOS:4-22). The α-amylase gene mutations encoding for these changes were made by PCR, according to the procedure summarized in Figure 9, using the PCR primers shown in Figure 2 (SEQ ID NOS:23-32).

Step (1): The mutagenic primers were used as templates for the PCR primers PCR A+ and PCR B- resulting in a lengthened (61 bp) double stranded DNA. Each contained a different amino acid replacement at position 188, and all except N188M contained a different restriction site. Initially the PCR primers were annealed at 35° C for five minutes followed by a one minute DNA extension with taq polymerase at 75° C. The double stranded DNA was then melted at 95° C for one minute, followed by the annealing and extension steps. Melting, annealing and extension continued for a total of 30 cycles.

Step (2): DNA upstream and downstream of position 188 were made in separate PCR reactions. The template was pBLapr, and the PCR primers were LAAfs5 (SEQ ID NO:27) and PCR A- (SEQ ID NO:24) for upstream; and PCR B+ (SEQ ID NO:25) and PCR Cla-Sall (SEQ ID NO:28) for downstream DNA. The DNA was melted at 95° C for one minute, annealed at 45° C for three minutes and elongated at 68° C for 3 minutes. The upstream portion is 290 bp and downstream is 498 bp. This procedure was repeated for 18 cycles using pfu polymerase. The same PCR procedure was used in steps (3) and (4).

Step (3): The upstream portion of DNA described in step (2) was attached to the double stranded mutagenic primers described in step (1). Primers LAAfs5 (SEQ ID NO:27) and PCR B- (SEQ ID NO:26) were used. As the result of primer design there is a 24 bp overlap between these templates allowing for the attachment of the two pieces of DNA.

Step (4): The downstream portions of DNA described in Step (2) and the product of Step (3) were attached to give the final product. A 24 bp overlap between the two PCR products allows for the attachment. Primers used were LAAfs5 (SEQ ID NO:27) and PCR Clal-Sall (SEQ ID NO:28).

Step (5): Unique restriction sites, Asp718 and BssHII, are located upstream and downstream, respectively, of the 188 site. The final PCR product Is digested with Asp718 and BssHII, the 333 bp fragment isolated by polyacrylamide gel electrophoresis and subcloned into the pHP.BL vector to obtain pHP.N188X.

Mutations were confirmed by dideoxy sequencing (Sanger et al., Proc. Natl. Acad. Sci. U.S.A., vol. 74, pp. 5463-5487 (1977)).

With reference to the DNA sequence and numbering system used in Figure 3, the codon encoding for the +188 amino acid position is at base pairs 812-814. PCR primers A+ and A- correspond to base pairs 784-807. PCR primers B+ and B- correspond to base pairs 821-844. The 5' end of PCR primer LAAfs5 corresponds to base pair 518. The 5' end of PCR primer PCR Clal-Sell corresponds to base pair 1317. The Asp718 site corresponds to base pair 724. The BssHII site corresponds to base pair 1053.

Reference EXAMPLE 3

Construction Of Plasmid Encoring Mutations At M15 And N188

A pBLapr plasmid having threonine substituted for methionine at amino acid 15 was constructed according to U.S. Patent Application Serial No. 08/194,664 (PCT Publication No. WO 94/18314). This plasmid (pBLaprM15T) was digested with Sfil and Asp718, and the 477 base pair fragment subcloned into pHP.BL to create pHP.M15T. In a manner analogous to that described above, Example 1, pHP.M15T was digested with Asp718 and BssHII, gel purified and eluted from the gel. The 333 base pair fragment comprising Asp718 to BssHII and the fragment from pHP.N188S were then subcloned into pHP.M15T to give plasmid pHP.M15T/N188S. In an analogous manner, starting with plasmids pBL aprM15L and pHP.NI88Y, the plasmid pHP.M15L/N188Y was constructed.

Reference EXAMPLE 4

Transformation Of Plasmids Into *Bacillus subtilis.* Expression And Purification of Mutant α-Amylase

α-Amylase was expressed in *Bacillus subtilis* after transformation with the plasmids described in Examples 1-3. pH13 is a plasmid able to replicate in *E. coll* and in *Bacillus subtills.* Plasmids containing different variants were constructed using *E. coil* strain MM294, the plasmids isolated and then transformed into *Bacillus subtilis* as described in Anagnostopoulos et al., J. Bacter., vol. 81, pp. 741-746 (1961). The *Bacillus* strain had been deleted for two proteases ($\Delta$epr, $\Delta$npr) (see e.g., Ferrari at al., U.S. Patent No. 5,264,366) and for amylase ($\Delta amyE$) (see e.g., Stahl et al., J. Bacter., vol. 158, pp. 411-418 (1984)). The *bacillus* strain expressing M15UN188Y was found to form larger zones of clearing than the strain expressing M15L on agar plates containing 1% insoluble starch indicating increased amylolytic activity. After transformation, the sacU(Hy) mutation (Henner et al., J. Bacter., vol., 170, pp. 296-300 (1988)) was introduced by PBS-1 mediated transduction (Hoch, J. Bact., vol. 154, pp. 1513-1515 (1983)).

Secreted amylases were routinely recovered from *Bacillus subtilis* cultures as follows: The culture supernatant was adjusted to 20% saturated ammonium sulfate and stirred for one hr. at 4°C. After centrifugation, the resultant supernatant was adjusted to 70% saturated ammonium sulfate and stirred for one hr. at 4°C. After centrifugation of the supernatant, the resultant pellet was re-dissolved in 50mM sodium acetate, pH 6.0, 5mM calcium chloride, and sterile filtered.

Reference EXAMPLE 5

Assay For Determining $\alpha$-Amylass Activity

Soluble Substrate Assay: A rate assay was developed based on an end-point assay kit supplied by Megazyme (Aust.) Pty. Ltd. A vial of substrate (*p*-nitrophenyl maltoheptaoside, BPNPG7) was dissolved in 10ml of sterile water followed by a 1:4 dilution in assay buffer (50mM maleate buffer, pH 6.7, 5mM calcium chloride, 0.002% Tween20). Assays were performed by adding 10µl of amylase to 790µl of the substrate in a cuvette at 25°C. Rates of hydrolysis were measured as the rate of change of absorbance at 410nm, after a delay of 75 seconds. The assay was linear up to rates of 0.2 absorption units/min.

$\alpha$-Amylase protein concentration was measured using the standard Bio-Rad Assay (Bio-Rad Laboratories) based on the method of Bradford, Anal. Biochem., vol. 72, p. 248 (1976) using bovine serum albumin standards.

Starch Hydrolysis Assay: $\alpha$-Amylase activity on starch was determined through an assay which depends on the ability of starch to form a blue colored complex with iodine and the disappearance of this color when starch is hydrolyzed to shorter dextrin molecules. The $\alpha$-amylase activity was defined in terms of the digestion time required to produce a color change denoting a definite state of dextrination of the starch.

Reagents used were as follows:

*Phosphate buffer* - Potassium dihydrogen phosphate (340 g) and sodium hydroxide (25.3 g) were dissolved in water and diluted to - two liters. The buffer was cooled to room temperature and the pH was adjusted to 6.2$\pm$0.1. The buffer was diluted to two liters in a volumetric flask.

*Starch substrate* - Ten grams (dry substance) of soluble lintner starch were suspended in 50 ml of water and washed into ~300 ml of boiling water. The suspension was again brought to boiling and was boiled for five minutes with constant stirring. The starch solution was cooled with constant stirring to room temperature and 125 ml of phosphate buffer was added. The solution was diluted to 500 ml with water. The starch substrate was made fresh daily.

*Stock iodine solution* - Iodine crystals (5.5 g) and potassium iodide (11.0 g) were dissolved in water and were volumetrically diluted to 250 ml. The solution was kept from light.

*Dilute iodine solution* - Potassium iodide (20 g) and two ml of stock iodine solution were dissolved in water and diluted volumetrically to 500 ml. The solution was made fresh daily.

*Enzyme diluting solution* - Calcium chloride (11.1 g) was dissolved in four liters of water. Water used for all reagents was either distilled or deionized.

An $\alpha$-amylase sample was diluted to between 10-15 LU/ml (as defined below) with enzyme diluting solution. For many commercial $\alpha$-amylase preparations a suitable dilution was found to be 2000 fold. Five milliliter aliquots of dilute iodine solution were dispensed into 13 x 100 mm test tubes and 10 ml of starch substrate was placed in a 23 x 200 mm test tube. All tubes were placed in the 30°C water bath. A Hellige comparator equipped with a special $\alpha$-amylase color disc (catalog number 620-s5) was used to make readings. Five milliliters of diluted enzyme (also at 30°C) were mixed with the starch substrate and timing was begun. At appropriate time intervals, for example one minute intervals early in the reaction and 15 second intervals later in the reaction, one ml aliquots of the enzyme-substrate mixture were transferred to a tube containing the dilute iodine solution. The starch iodine solution was mixed and transferred to a 13 mm precision square tube and the color was compared with the standard $\alpha$-amylase color disc in the Heilige comparator. When the time of the end point was approached, samples were taken at 0.25 minute intervals.

The time required for the colors of the samples and the color disc to match were recorded and the activity (in liquefons per gram or ml) was calculated according to the formula:

## LU/ml or LU/g =

Where:LU = liquefon unit
V = volume of enzyme (5 ml or grams)
t = dextrinization time (minutes)
D = dilution factor:dilution volume divided by ml or g of enzyme diluted.

Reference Example 6

Preparation and Testing of Additional Mutant Alpha-Amylases for Thermal Stability

*Bacillus stearothermophilus* and *Bacillus amyloliquefaciens* produce an α-amylase which has poorer stability than the α-amylase produced by *Bacillus licheniformis*. From an alignment of these amylases, residues which differ in *B. stearothermophilus* or *B. amylollquefaciens* compared to *B. lichaniformis* were identified. From this analysis, mutant α-amylases based on the *B. lichenifomis* sequence were prepared having substitutions at one or more of five positions for which corresponding residues in both *Bacillus stesrothermophllus* and *Bacillus emyloliquefactens* were identical but differed from *B. licheniformis*: A33S, A52S, N96Q S148N, A379S in combination with M15T/H133Y/N1885/A209V and compared with a mutant comprising only the M15T/H133Y/N1885/A209V substitutions. Additionally, the mutation S85D was incorporated which represents a recruitment only from the *Bacillus emylotiquefaciens* α-amylase. The mutations were prepared according to the procedures provided in Examples 1-4 except that appropriate PCR primers were provided to effect the desired mutations.

Thermal inactivation rates for the various mutants were measured according to the following procedure. Amylase stock solutions were dlalysed extensively Into 20 mM ammonium acetate, 4 mM $CaCl_2$ pH 6.5. For measurement of stability, this stock was diluted >50fold into a solution designed to induce rapid inactivation of wild type amylase: 50mM ammonium acetate, 5mM $CaCl_2$, to Tween 20 and a pH of 4.9, or 4.8 to a final concentration of between 30 and 50 μg/ml. Six 100μl aliquots were put into Eppendorf tubes and placed into a water bath at either 82°C or 830°C. The Eppendorf tubes were

removed at regular, measured intervals of between 30 seconds and 5 minutes and placed on ice to stop the inactivation. The residual activity was assayed using a soluble substrate as described in Example 5. The natural log of the activity was plotted against time of incubation, and the rate constant for inactivation obtained from the slope of the straight line. The half-life was calculated as *In*(2) divided by the rate constant. Results for various mutants are provided in Tables 1-4.

### TABLE 1

| pH 4.9, 83°C | | |
|---|---|---|
| Variant | Inactivation rate constant (Min$^{-1}$) | $t_{1/2}$ (min.) |
| M15T/H133Y/N188S/A209V | 0.171 | 4.05 |
| M15T/D28N/A33S/H133Y/N188S/A209V | 0.137 | 5.06 |
| M15T/A52S/H133Y/N188S/A209V/L230F | 0.144 | 4.81 |
| M15T/N96Q/H133Y/N188S/A209V/I479T | 0.162 | 4.27 |
| M15T/H133Y/S148N/N188S/A209V/G433D | 0.121 | 5.73 |
| M15T/H133Y/N188S/A209V/A379S | 0.145 | 4.78 |

### TABLE 2

| pH 4.85, 83°C | | |
|---|---|---|
| Variant | Inactivation rate constant (Min$^{-1}$) | $t_{1/2}$ (min.) |
| M15T/H133Y/N188S/A209V | 0.252 | 2.75 |
| M15T/H133Y/N188S/A209V | 0.235 | 2.95 |
| M15T/H133Y/S148N/N188S/A209V/A379S | 0.114 | 6.05 |

### TABLE 3

| pH4.85, 82°C | | |
|---|---|---|
| Variant | Inactivation rate constant (Min$^{-1}$) | $t_{1/2}$ (min.) |
| M15T/H133Y/N188S/A209V | 0.203 | 3.41 |
| M15T/H133Y/S148N/N188S/A209V/A379S | 0.106 | 6.54 |
| M15T/H133Y/N188S/A209V/A210ST322A/A379S | 0.141 | 4.89 |
| M15T/H133Y/S148N/N188S/A209V | 0.122 | 5.65 |

### TABLE 4

| pH4.80, 82.2°C | | |
|---|---|---|
| Variant | Inactivation rate constant (Min$^{-1}$) | $t_{1/2}$ (min.) |
| Wild Type | >2.0 | <0.35 |
| M15T/N188S | >1.9 | <0.36 |
| M15T/H133Y/N188S/A209V | 0.267 | 2.59 |
| M15T/S85D/H 133Y/N188S/A209V | 0.236 | 2.93 |

EXAMPLE 7

Recruitment of Residues From Less Stable Cellulases to More Stable Cellulases for Improvement of Stability of the More Stable Cellulase

Homologues of *Trichoderma reesei* EGIII cellulase from various species were obtained and tested for thermal stability. The cellulases were obtained from *(A) Hypocrea schweinitzii, (B)Aspergillus aculeatus, (C) Aspergillus kawachii, (D)Gliocladium roseum (1),(E) Gliocladium roseum* (2), *(F) Gliocladium roseum* (3)and *(G) Humicola grisea*. Of the above, cellulases (a) through (f) are less thermally stable than EGIII under the conditions of the assay. Cellulase (g) is more stable than EGIII. Accordingly, from the aligned sequences of these cellulases (as provided in Figure 11), it is possible to pick out the residues which exist in the less stable cellulase as compared to the more stable cellulase. To simplify reading of the table, positions marked for mutation are highlighted in bold. Mutants of EGIII were then prepared based on this information. In addition, mutant cellulase (g) was prepared based on recruitments from EGIII.

Equilibrium circular dichroism experiments were performed on an Aviv 62DS or 62ADS spectrophotometer, equipped with a 5 position thermoelectric cell holder supplied by Aviv. Buffer conditions were 50 mM bis-tris propane and 50 mM ammonium acetate adjusted to pH 8.0 with acetic acid. The final protein concentration for each experiment was in the range of 5-30 µM. Data was collected in a 0.1 cm path length cell.

Spectra were collected from 265-~10 nm. Thermal denaturations were performed at 217 nm from 30 to 90°C with data collected every two degrees. The equilibration time at each temperature was 0.1 minutes and data was collected for 4 seconds per sample.

The remainder of the pH 8.0 sample was divided into 5 x 400 uL aliquots. Two samples were adjusted to pH 5 and 7 with acetic acid and two others were adjusted to pH 9 and 10 with sodium hydroxide. Thermal denaturations of all samples were performed simultaneously as described above.

Table 5 illustrates 24 mutants which were prepared based on recruitments from less stable cellulases into EGIII and 3 mutants which were prepared based on recruitments from EGIII into a homolog isolated from *Humicola grisea* (note that the numbering in Table 5 for H.grisea is based on EGIII numbering in Figure 11). Of these, a significant number had improved stability over the wild type molecule (either EGIII or cellulases (g)) as shown in bold in Table 5.

### TABLE 5

| | Mutation | $\Delta T_m$ | $T_m$ (°C) | Fit error |
|---|---|---|---|---|
| PR | Product | | 54.10 | 0.09 |
| 0 | WT EG3 | | 54.60 | 0.18 |

(continued)

|   | Mutation | $\Delta T_m$ | $T_m$ (°C) | Fit error |
|---|----------|------|------|-----------|
| 1 | W7Y | -1.03 | 53.40 | 0.24 |
| 2 | **T11S/T16I** | 1.07 | 55.50 | 0.13 |
| 3 | A35S | -4.03 | 50.40 | 0.14 |
| 4 | **S39N** | 0.47 | 54.90 | 0.17 |
| 5 | **G41A** | 2.47 | 56.90 | 0.11 |
| 6 | S63V | -0.83 | 53.60 | 0.11 |
| 7 | A66N | 0.07 | 54.50 | 0.10 |
| 8 | S77G | 0.07 | 54.50 | 0.09 |
| 9 | **N91D** | 0.47 | 54.90 | 0.17 |
| 10 | **S143T** | 0.47 | 54.90 | 0.12 |
| 11 | **T163S** | 0.27 | 54.70 | 0.07 |
| 12 | **N167S** | 0.17 | 54.60 | 0.10 |
| 13 | **A188G** | 0.47 | 54.90 | 0.17 |
| 14 | G31Q | -14.03 | 40.40 | 0.15 |
| 15 | Q162P | 0.07 | 54.50 | 0.19 |
| 16 | Y168F | -0.03 | 54.40 | 0.12 |
| 17 | **N174D** | 1.17 | 55.60 | 0.44 |
| 18 | V192L | -0.23 | 54.20 | 0.13 |
| 19 | N164D | -2.33 | 52.10 | 0.33 |
| 20 | **P201C** | 3.9/17.4 | 58.3/71.8 | .15/.23 |
| 21 | **V210C** | 0.47 | 70.60 | 70.80 |
| 22 | Q162P/T166P (17a* R2-2) | -1.43 | 53.00 | 0.29 |
| 23 | M79I | -7.13 | 47.30 | 0.27 |
| 24 | **T145E/Y147W** (25a4) | 0.77 | 55.20 | 0.05 |
| G0 | WT H. greisii | 0.00 | 68.5 | 0.11 |
| G1 | **C210V** | 1.50 | 70 | 0.2 |
| G2 | **C170G** | 2.10 | 70.6 | 0.23 |

## Claims

1. An improved cellulase comprising an amino acid sequence which has been modified from a precursor amino acid sequence having the sequence of *T. reesei* EGIII cellulase shown in Figure 11, said modification comprising a substitution P201C, said amino acid numbering being as in Figure 11; wherein the improved cellulase has increased thermostability compared to a cellulase having the precursor amino acid sequence.

2. A DNA sequence encoding a cellulase according to claim 1.

3. An expression vector comprising a DNA sequence according to claim 2.

4. A host cell comprising a DNA sequence according to claim 2 or an expression vector according to claim 3.

5. A method of producing a cellulase according to claim 1, comprising:

(a) modifying a DNA sequence encoding the precursor amino acid sequence to produce a.modified DNA sequence as defined in claim 2 encoding the improved cellulase;

(b) expressing the modified DNA sequence in a host cell; and

(c) purifying the expressed cellulase protein.

**6.** Use of a cellulase according to claim 1 in biomass reduction, cleaning products or textile treatment compositions.

**Patentansprüche**

**1.** Verbesserte Cellulase, umfassend eine Aminosäuresequenz, die aus einer Vorläufer-Aminosäuresequenz, die die in Figur 11 dargestellte Sequenz von T.-reesei-EGIII-Cellulase aufweist, modifiziert wurde, welche Modifizierung eine Substitution P201C umfasst, wobei die Aminosäurenummerierung wie in Figur 11 gilt; worin die verbesserte Cellulase im Vergleich zu einer Cellulase mit der Vorläufer-Aminosäuresequenz eine erhöhte Wärmestabilität aufweist.

**2.** DNA-Sequenz, kodierend für eine Cellulase nach Anspruch 1.

**3.** Expressionsvektor, umfassend eine DNA-Sequenz nach Anspruch 2.

**4.** Wirtszelle, umfassend eine DNA-Sequenz nach Anspruch 2 oder einen Expressionsvektor nach Anspruch 3.

**5.** Verfahren zur Herstellung einer Cellulase nach Anspruch 1, umfassend:

(a) das Modifizieren einer DNA-Sequenz, die für die Vorläufer-Aminosäuresequenz kodiert, um eine modifizierte DNA-Sequenz nach Anspruch 2 zu produzieren, die für die verbesserte Cellulase kodiert;

(b) das Exprimieren der modifizierten DNA-Sequenz in einer Wirtszelle; und

(c) das Reinigen des exprimierten Cellulaseproteins.

**6.** Verwendung einer Cellulase nach Anspruch 1 bei der Biomassereduktion, in Reinigungsprodukten oder in Zusammensetzungen zur Textilienbehandlung.

**Revendications**

**1.** Cellulase améliorée comprenant une séquence d'acides aminés qui a été modifiée à partir d'une séquence d'acides aminés précurseur ayant la séquence de *T.reesei* EGIII cellulase indiquée dans la Figure 11, ladite modification comprenant une substitution P201C, ladite numérotation des d'acides aminés étant comme sur la Figure 11; où la cellulase améliorée a une plus grande thermostabilité comparée à une cellulase ayant la séquence d'acides aminés précurseur.

**2.** Séquence d'ADN codant pour une cellulase selon la revendication 1.

**3.** Vecteur d'expression comprenant une séquence d'ADN selon la revendication 2.

**4.** Cellule hôte comprenant une séquence d'ADN selon la revendication 2 ou un vecteur d'expression selon la revendication 3.

**5.** Méthode de production d'une cellulase selon la revendication 1, comprenant:

(a) modifier une séquence d'ADN codant pour la séquence d'acides aminés précurseur pour produire une séquence d'ADN modifiée telle que définie dans la revendication 2 codant pour la cellulase améliorée;

(b) exprimer la séquence d'ADN modifiée dans une cellule hôte; et

(c) purifier la protéine de cellulase exprimée.

**6.** Utilisation d'une cellulase selon la revendication 1 dans la réduction d'une biomasse, dans des produits de nettoyage ou des compositions de traitement textile.

188

N188T  5'-G GAT TGG GAA GT<u>G TCG ACT</u> GAA AAC GGC AAC TAT GAT-3'  (SEQ ID NO:4)
Sall

N188P  5'-G GAT TGG GAA GTT TCC <u>CCA GAA AAT GGC</u> AAC TAT GAT-3'  (SEQ ID NO:5)
pflMI

N188R  5'-G GAT TGG GAA GTT <u>TCT AGA</u> GAA AAC GGC AAC TAT GAT-3'  (SEQ ID NO:6)
Xbal

N188L  5'-G GAT TGG GAA GTT TCC <u>CTC GAG</u> AAC GGC AAC TAT GAT-3'  (SEQ ID NO:7)
Xhol

N188A  5'-G GAT TGG GAA GTT TC<u>G GCC GAA</u> AAC GGC AAC TAT GAT-3'  (SEQ ID NO:8)
Eagl

N188G  5'-G GAT TGG GAA GTT TC<u>C GGA</u> GAA AAC GGC AAC TAT GAT-3'  (SEQ ID NO:9)
BspEl

N188V  5'-G GAT TGG GAA GTT AG<u>C GTC</u> GAA AAC GGC AAC TAT GAT-3'  (SEQ ID NO:10)
Hgal

N188K  5'-G GAT TGG GAA GTT <u>TCC AAG</u> GAA AAC GGC AAC TAT GAT-3'  (SEQ ID NO:11)
Styl

N188Q  5'-G GAT TGG GAA GTT TCC <u>CAG GAA AAT</u> GGC AAC TAT GAT-3'  (SEQ ID NO:12)
BstXI

N188H  5'-G GAT TGG GAA GTT <u>TCT CAT</u> GAA AAC GGC AAC TAT GAT-3'  (SEQ ID NO:13)
BspHI

## FIG._1A

EP 1 240 524 B1

188

N188E 5'-G GAT TGG GAA GTT TCC GAA GAG AAC GGC AAC TAT GAT-3' (SEQ ID NO:14)
EarI

N188D 5'-G GAT TGG GAA GTT TCC GAG GAG AAC GGC AAC TAT GAT-3' (SEQ ID NO:15)
BseRI

N188Y 5'-G GAT TGG GAA GTT TCA TAT GAA AAC GGC AAC TAT GAT-3' (SEQ ID NO:16)
NdeI

N188C 5'-G GAT TGG GAA GTC TCC TGC GAA AAC GGC AAC TAT GAT-3' (SEQ ID NO:17)
BsmAI

N188F 5'-G GAT TGG GAA GTT TCC TTC GAA AAC GGC AAC TAT GAT-3' (SEQ ID NO:18)
BstBI

N188I 5'-G GAT TGG GAA GTT TCG ATC GAA AAC GGC AAC TAT GAT-3' (SEQ ID NO:19)
PvuI

N188M 5'-G GAT TGG GAA GTT TCC ATG GAA AAC GGC AAC TAT GAT-3' (SEQ ID NO:20)

N188w 5'-G GAT TGG GAA GTT TCC TGG GAA AAC GGC AAC TAT GAT-3' (SEQ ID NO:21)
BstNI

N188S 5'-G GAT TGG GAA GTG AGC TCT GAA AAC GGC AAC TAT GAT-3' (SEQ ID NO:22)
SstI

*FIG._1B*

EP 1 240 524 B1

|  |  | 179 |  |
|---|---|---|---|
| PCR A+ | | 5'-AGG AAA GGC TTG GGA TTG GGA AGT-3' | (SEQ ID NO:23) |

|  |  | 179 |  |
|---|---|---|---|
| PCR A– | | 5'-ACT TCC CAA TCC CAA GCC TTT CCT-3' | (SEQ ID NO:24) |

|  |  | 191 |  |
|---|---|---|---|
| PCR B+ | | 5'-GGC AAC TAT GAT TAT TTG ATG TAT-3' | (SEQ ID NO:25) |

|  |  | 191 |  |
|---|---|---|---|
| PCR B– | | 5'-ATA CAT CAA ATA ATC ATA GTT GCC-3' | (SEQ ID NO:26) |

|  |  | 90 |  |
|---|---|---|---|
| PCR LAAfs5 | | 5'-CTT CAT TCC CGC GAC ATT AAC-3' | (SEQ ID NO:27) |

|  |  | 356 |  |
|---|---|---|---|
| PCR ClaI-SalI | | 5'-GA TTC CCT TGT GAG AAT AAA AG-3' | (SEQ ID NO:28) |

|  |  | 246 |  |
|---|---|---|---|
| PCR I+ | | 5'-AAT CAT GTC AGG GAA AAA ACT GGG-3' <br> Bsrl | (SEQ ID NO:29) |

|  |  | 246 |  |
|---|---|---|---|
| PCR I– | | 5'-CCC AGT TTT TTC CCT GAC ATG ATT-3' <br> Bsrl | (SEQ ID NO:30) |

|  |  | 257 |  |
|---|---|---|---|
| PCR J+ | | 5'-TTT ACG GTA GCT GAA TAT TGG CAG-3' | (SEQ ID NO:31) |

|  |  | 257 |  |
|---|---|---|---|
| PCR J– | | 5'-CTG CCA ATA TTC AGC TAC CGT AAA-3' | (SEQ ID NO:32) |

**FIG._2**

EP 1 240 524 B1

```
AGCTTGAAGA AGTGAAGAAG CAGAGAGGCT ATTGAATAAA TGAGTAGAAA GCGCCATATC          60


GGCGCTTTTC TTTTGGAAGA AAATATAGGG AAAATGGTAC TTGTTAAAAA TTCGGAATAT         120


TTATACAACA TCATATGTTT CACATTGAAA GGGGAGGAGA ATCATGAAAC AACAAAAACG         180
                                                 M   K   Q    Q   K   R

GCTTTACGCC CGATTGCTGA CGCTGTTATT TGCGCTCATC TTCTTGCTGC CTCATTCTGC         240
 L   Y   A   R   L   L   T    L   L   F   A   L   I    F   L   L   P   H   S   A

AGCAGCGGCG GCAAATCTTA ATGGGACGCT GATGCAGTAT TTTGAATGGT ACATGCCCAA         300
 A   A   A   A   N   L   N    G   T   L   M   Q   Y    F   E   W   Y   M   P   N

TGACGGCCAA CATTGGAAGC GTTTGCAAAA CGACTCGGCA TATTTGGCTG AACACGGTAT         360
 D   G   Q   H   W   K   R    L   Q   N    D   S  A    Y   L   A   E   H   G   I

TACTGCCGTC TGGATTCCCC CGGCATATAA GGGAACGAGC CAAGCGGATG TGGGCTACGG         420
 T   A   V   W   I   P   P    A   Y   K    G   T   S    Q   A   D   V    G   Y   G

TGCTTACGAC CTTTATGATT TAGGGGAGTT TCATCAAAAA GGGACGGTTC GGACAAAGTA         480
 A   Y   D   L   Y   D   L    G   E   F    H   Q   K    G   T  V   R    T   K   Y

CGGCACAAAA GGAGAGCTGC AATCTGCGAT CAAAAGTCTT CATTCCCGCG ACATTAACGT         540
 G   T   K   G   E   L   Q    S   A   I    K   S   L    H   S   R   D    I   N   V

TTACGGGGAT GTGGTCATCA ACCACAAAGG CGGCGCTGAT GCGACCGAAG ATGTAACCGC         600
 Y   G   D   V   V   I   N    H   K   G    G   A   D    A   T   E   D    V   T   A

GGTTGAAGTC GATCCCGCTG ACCGCAACCG CGTAATTTCA GGAGAACACC TAATTAAAGC         660
 V   E   V   D   P   A   D    R   N   R    V   I   S    G   E   H   L    I   K   A
```

FIG._3A

```
CTGGACACAT  TTTCATTTTC  CGGGGCGCGG  CAGCACATAC  AGCGATTTTA  AATGGCATTG      720
 W  T  H     F  H  F  P    G  R  G     S  T  Y    S  D  F  K    W  H  W

GTACCATTTT  GACGGAACCG  ATTGGGACGA  GTCCCGAAAG  CTGAACCGCA  TCTATAAGTT      780
 Y  H  F     D  G  T  D    W  D  E     S  R  K    L  N  R  I    Y  K  F

TCAAGGAAAG  GCTTGGGATT  GGGAAGTTTC  CAATGAAAAC  GGCAACTATG  ATTATTTGAT      840
 Q  G  K     A  W  D  W    E  V  S     N  E  N    G  N  Y  D    Y  L  M

GTATGCCGAC  ATCGATTATG  ACCATCCTGA  TGTCGCAGCA  GAAATTAAGA  GATGGGGCAC      900
 Y  A  D     I  D  Y  D    H  P  D     V  A  A    E  I  K  R    W  G  T

TTGGTATGCC  AATGAACTGC  AATTGGACGG  TTTCCGTCTT  GATGCTGTCA  AACACATTAA      960
 W  Y  A     N  E  L  Q    L  D  G     F  R  L    D  A  V  K    H  I  K

ATTTTCTTTT  TTGCGGGATT  GGGTTAATCA  TGTCAGGGAA  AAAACGGGGA  AGGAAATGTT     1020
 F  S  F     L  R  D  W    V  N  H     V  R  E    K  T  G  K    E  M  F

TACGGTAGCT  GAATATTGGC  AGAATGACTT  GGGCGCGCTG  GAAAACTATT  TGAACAAAAC     1080
 T  V  A     E  Y  W  Q    N  D  L     G  A  L    E  N  Y  L    N  K  T

AAATTTTAAT  CATTCAGTGT  TTGACGTGCC  GCTTCATTAT  CAGTTCCATG  CTGCATCGAC     1140
 N  F  N     H  S  V  F    D  V  P     L  H  Y    Q  F  H  A    A  S  T

ACAGGGAGGC  GGCTATGATA  TGAGGAAATT  GCTGAACGGT  ACGGTCGTTT  CCAAGCATCC     1200
 Q  G  G     G  Y  D  M    R  K  L     L  N  G    T  V  V  S    K  H  P

GTTGAAATCG  GTTACATTTG  TCGATAACCA  TGATACACAG  CCGGGGCAAT  CGCTTGAGTC     1260
 L  K  S     V  T  F  V    D  N  H     D  T  Q    P  G  Q  S    L  E  S

GACTGTCCAA  ACATGGTTTA  AGCCGCTTGC  TTACGCTTTT  ATTCTCACAA  GGGAATCTGG     1320
 T  V  Q     T  W  F  K    P  L  A     Y  A  F    I  L  T  R    E  S  G
```

*FIG._3B*

EP 1 240 524 B1

```
ATACCCTCAG GTTTTCTACG GGGATATGTA CGGGACGAAA GGAGACTCCC AGCGCGAAAT    1380
 Y   P   Q   V   F   Y   G   D   M   Y   G   T   K   G   D   S   Q   R   E   I

TCCTGCCTTG AAACACAAAA TTGAACCGAT CTTAAAAGCG AGAAAACAGT ATGCGTACGG    1440
 P   A   L   K   H   K   I   E   P   I   L   K   A   R   K   Q   Y   A   Y   G

AGCACAGCAT GATTATTTCG ACCACCATGA CATTGTCGGC TGGACAAGGG AAGGCGACAG    1500
 A   Q   H   D   Y   F   D   H   H   D   I   V   G   W   T   R   E   G   D   S

CTCGGTTGCA AATTCAGGTT TGGCGGCATT AATAACAGAC GGACCCGGTG GGGCAAAGCG    1560
 S   V   A   N   S   G   L   A   A   L   I   T   D   G   P   G   G   A   K   R

AATGTATGTC GGCCGGCAAA ACGCCGGTGA GACATGGCAT GACATTACCG GAAACCGTTC    1620
 M   Y   V   G   R   Q   N   A   G   E   T   W   H   D   I   T   G   N   R   S

GGAGCCGGTT GTCATCAATT CGGAAGGCTG GGGAGAGTTT CACGTAAACG GCGGGTCGGT    1680
 E   P   V   V   I   N   S   E   G   W   G   E   F   H   V   N   G   G   S   V

TTCAATTTAT GTTCAAAGAT AGAAGAGCAG AGAGGACGGA TTTCCTGAAG GAAATCCGTT    1740
 S   I   Y   V   Q   R   *

TTTTTATTTT GCCCGTCTTA TAAATTTCTT TGATTACATT TTATAATTAA TTTTAACAAA    1800

GTGTCATCAG CCCTCAGGAA GGACTTGCTG ACAGTTTGAA TCGCATAGGT AAGGCGGGGA    1860

TGAAATGGCA ACGTTATCTG ATGTAGCAAA GAAAGCAAAT GTGTCGAAAA TGACGGTATC    1920

GCGGGTGATC AATCATCCTG AGACTGTGAC GGATGAATTG AAAAAGCT                 1968
```

## FIG._3C

```
ANLNGTLMQY  FEWYMPNDGQ  HWKRLQNDSA  YLAEHGITAV  WIPPAYKGTS  QADVGYGAYD       60

LYDLGEFHQK  GTVRTKYGTK  GELQSAIKSL  HSRDINVYGD  VVINHKGGAD  ATEDVTAVEV      120

DPADRNRVIS  GEHLIKAWTH  FHFPGRGSTY  SDFKWHWYHF  DGTDWDESRK  LNRIYKFQGK      180

AWDWEVSNEN  GNYDYLMYAD  IDYDHPDVAA  EIKRWGTWYA  NELQLDGFRL  DAVKHIKFSF      240

LRDWVNHVRE  KTGKEMFTVA  EYWQNDLGAL  ENYLNKTNFN  HSVFDVPLHY  QFHAASTQGG      300

GYDMRKLLNG  TVVSKHPLKS  VIFVDNHDTQ  PGQSLESTVQ  TWFKPLAYAF  ILTRESGYPQ      360

VFYGDMYGTK  GDSQREIPAL  KHKIEPILKA  RKQYAYGAQH  DYFDHHDIVG  WTREGDSSVA      420

NSGLAALITD  GPGGAKRMYV  GRQNAGETWH  DITGNRSEPV  VINSEGWGEF  HVNGGSVSIY      480

VQR
```

*FIG._4*

EP 1 240 524 B1

Am-Lich  = B.licheniformis    Am-Amylo = B.amyloliquefaciens    Am-Stero = B.stearothermophilus.

```
                   1                                                      1
Am-Lich      ......MKQQ KRLYARLLTL LFALIFLLPH ......SAAA AANLNGTLMQ YFEWYMPNDG   19
Am-Amylo     MRGRGNMIQK RKRTVSFRLV LMCTLLFVSL ......PITK TSAVNGTLMQ YFEWYTPNDG   60
Am-Stearo    ......VLTF HRIIRKGWMF LLAFLLTASL FCPTGRHAKA AAPFNGTMMQ YFEWYLPDDG

                  61
Am-Lich      QHWKRLQNDS AYLAEHGITA VWIPPAYKGT SQADVGYGAY DLYDLGEFHQ KGTVRTKYGT   79
Am-Amylo     QHWKRLQNDA EHLSDIGITA VWIPPAYKGL SQSDNGYGPY DLYDLGEFQQ KGTVRTKYGT   120
Am-Stearo    TLWTKVANEA NNLSSLGITA LSLPPAYKGT SRSDVGYGVY DLYDLGEFNQ KGTVRTKYGT

                 121
Am-Lich      KGELQSAIKS LHSRDINVYG DVVINHKGGA DATEDVTAVE VDPADRNRVI SGEHLIKAWT   139
Am-Amylo     KSELQDAIGS LHSRNVQVYG DVVLNHKAGA DATEDVTAVE VNPANRNQET SEEYQIKAWT   180
Am-Stearo    KAQYLQAIQA AHAAGMQVYA DVVFDHKGGA DGTEWVDAVE VNPSDRNQEI SGTYQIQAWT

                 181
Am-Lich      HFHFPGRGST YSDFKWHWYH FDGTDWDESR KLNRIYKF.. QGKAWDWEVS NENGNYDYLM   197
Am-Amylo     DFRFPGRGNT YSDFKWHWYH FDGADWDESR KISRIFKFRG EGKAWDWEVS SENGNYDYLM   240
Am-Stearo    KFDFPGRGNT YSSFKWRWYH FDGVDWDESR KLSRIYKFRG IGKAWDWEVD TENGNYDYLM

                 241
Am-Lich      YADIDYDHPD VAAEIKRWGT WYANELQLDG FRLDAVKHIK FSFLRDWVNH VREKTGKEMF   257
Am-Amylo     YADVDYDHPD VVAETKKWGI WYANELSLDG FRIDAAKHIK FSFLRDWVQA VRQATGKEMF   300
Am-Stearo    YADLDMDHPE VVTELKNWGK WYVNTTNIDG FRLDGLKHIK FSFFPDWLSY VRSQTGKPLF
```

*FIG._5A*

22

Am-Lich    = B.licheniformis    Am-Amylo = B.amyloliquefaciens    Am-Stero = B.stearothermophilus.

```
                                                                  317
           301                                                    360
Am-Lich    TVAEYWQNDL  GALENYLNKT  NFNHSVFDVP  LHYQFHAAST  QGGGYDMRKL  LNGTVVSKHP
Am-Amylo   TVAEYWQNNA  GKLENYLNKT  SFNQSVFDVP  LHFNLQAASS  QGGGYDMRRL  LDGTVVSRHP
Am-Stearo  TVGEYWSYDI  NKLHNYITKT  NGTMSLFDAP  LHNKFYTASK  SGGAFDMRTL  MTNTLMKDQP


                                                                  377
           361                                                    420
Am-Lich    LKSVTFVDNH  DTQPGQSLES  TVQTWFKPLA  YAFILTRESG  YPQVFYGDMY  GTKGDSQREI
Am-Amylo   EKAVTFVENH  DTQPGQSLES  TVQTWFKPLA  YAFILTRESG  YPQVFYGDMY  GTKGTSPKEI
Am-Stearo  TLAVTFVDNH  DTNPAKR.CS  HGRPWFKPLA  YAFILTRQEG  YPCVFYGDYY  GI...PQYNI


                                                                  437
           421                                                    480
Am-Lich    PALKHKIEPI  LKARKQYAYG  AQHDYFDHHD  IVGWTREGDS  SVANSGLAAL  ITDGPGGAKR
Am-Amylo   PSLKDNIEPI  LKARKEYAYG  PQHDYIDHPD  VIGWTREGDS  SAAKSGLAAL  ITDGPGGSKR
Am-Stearo  PSLKSKIDPL  LIARRDYAYG  TQHDYLDHSD  IIGWTREGVT  EKPGSGLAAL  ITDGAGRSKW


                                           483
           481                                                    540
Am-Lich    MYVGRQNAGE  TWHDITGNRS  EPVVINSEGW  GEFHVNGGSV  SIYVQR....  ..........
Am-Amylo   MYAGLKNAGE  TWYDITGNRS  DTVKIGSDGW  GEFHVNDGSV  SIYVQK....  ..........
Am-Stearo  MYVGKQHAGK  VFYDLTGNRS  DTVTINSDGW  GEFKVNGGSV  SVWVPRKTTV  STIARPITTR


           541                    559
Am-Lich    ..........  ........
Am-Amylo   ..........  ........
Am-Stearo  PWTGEFVRWH  EPRLVAWP*
```

*FIG._5B*

FIG._6

*FIG._7*

aprE — BLAA

HindIII    SfiI    Asp718    ClaI    SalI    EcoR1

(N188)    BssHII

NciI.4134    ORI 322    (lacI')    lacZ α    NcoI.477

HphI.3769    CmR    Ksp63.877

BclI.3376    pHP.BL
7330bp

EmR    Rep
pTA1060    NciI.1813

HpaI.3000    AsuII.1959
NsiI.2851
Sau96.2744
NciI.2686
NciI.2651
HgaI.2625

Key:    pC194
        pE194
        pUC9
        pTA1060
        pUB110

pHP.BL = pHP13 with the 2460bp HindIII-EcoRI insert from pBLapr

FIG._8

*FIG._9*

## SIGNAL SEQUENCE-MATURE PROTEIN JUNCTIONS IN:

*B.licheniformis*   alpha-amylase.                                    (PstI)

M K Q Q K R L T A R L L T L L F A L I F L L P H S A A A A **A N L** . . . . . . . . . .

*B.subtilis*   alkaline protease aprE.                          (PstI)

M R S K T L W I S L L F A L T L I F T M A F S N M S A Q A **A** G K S . . . . . . . . . . .

*B.licheniformis*   alpha-amylase in pBLapr.

M R S K T L W I S L L F A L T L I F T M A F S N M S A Q A **A N L** . . . . . . . . . . .

(PstI)      indicates the site of the restriction site in the gene

**BOLD TYPE** indicates the N-terminus of the secreted protein in *Bacillus*.

*FIG._10*

EP 1 240 524 B1

EP 1 240 524 B1

```
                                        1                                           17
        T._reesei  M.........KF.LQVLPALIPAALAQTS..............CDQWATFTGNG..YTV
   H._schweinitzii  M.........KF.LQVLPAILPAALAQTS.............CDQYATFSGNG..YIV
    A._aculeatus__* M.........KAFHL.LAALAGAAVAQQAQ...........LCDQYATYTGGV..YTI
     A._kawachii_2  M.........KAFHL.LAALSGAAVAQQAQ...........LCDQYATYTGGV..YTI
         H._grisei  M.......LKSALLLGAAAVSVQSASIPTIPANLEPRQIR.SLCELYGYWSGNG..YEL
      G._roseum_Rj_1 M..........KANIVILSLFAPLAAVAQT..............LCGQYSSNTQGG..YIF
      G._roseum_PA_3 M.........KFQLLSLTAFAPLSLAA...............LCGQYQSQSQGG..YIF
      G._roseum_Rj_4 M.........KTGIAYLAAVLPLA.MAES..............LCDQYAYLSRDG..YNF
                    18                                          63
        T._reesei  SNNLWGASAGSGF..GCV.TAVSLSGG.ASWHADWQWSGGQNNVKSYQNS..........
   H._schweinitzii  SNNLWGASAGSGF..GCV.TSVSLNGA.ASWHADWQWSGGQNNVKSYQNV..........
    A._aculeatus__* NNNLWGKDAGSG..SQCTTVNSASSAG.TSWSTKWNWSGGENSVKSYANS..........
     A._kawachii_2  NNNLWGKDAGSG..SQCTTVNSASSAG.TSWSTKWNWSGGENSVKSYANS..........
         H._grisei  LNNLWGKDTATS.GWQCTYLDGTNNGG.IQWNTAWEWQGAPDNVKNYPYV..........
      G._roseum_Rj_1 NNNMWGMGSGSGS..QCTYVDKVWAEG.VAWHTDWSWSGGDNNVKSYPYS..........
      G._roseum_PA_3 NNNKWGQGSGSGS..QCLTIDKTWDSN.VAFHADWSWSGGTNNVKSYPNA..........
      G._roseum_Rj_4 NNNEWGAATGTGD..QCTYVDSTSSGG.VSWHSDWTWSGSESEIKSYPYS..........
                    64                                          117
        T._reesei  .QIAIP.QKRTVNSISSMPTTASW...SYSGSNIRANVAYDL.FTAANPNHVTYSGDYEL
   H._schweinitzii  .QINIP.QKRTVNSIGSMPTTASW...SYSGSDIRANVAYDL..FTAANPNHVTYSGDYEL
    A._aculeatus__* .GLTF..NKKLVSQISQIPTTARW.S..YDNTGIRADVAYDL.FTAADINHVTWSGDYEL
     A._kawachii_2  .GLSF..NKKLVSQISHIPTAARW.S..YDNTCIRRGRAYDL.FTAADINHVTWSGDYEL
         H._grisei  .GKQIQRGRK.ISDINSMRTSVSW...TYDRTDLRANVAYDV.FTARDPDHPNWGGDYEL
      G._roseum_Rj_1 .GRELGT.KRIVSSIKSISSGADW...DYTGSNLRANAAYDI.FTSANPNHATSSGDYEV
      G._roseum_PA_3 .GLEFSR.GKKVSSIGTINGGADW...DYSGSNIRANVAYGI.FTSADPNHVTSSGDYEL
      G._roseum_Rj_4 .GLDLPE.KKIVTSIGSISTGAEW...SYSGSDIRADVAYDT.FTAADPNHATSSGDYEV
```

## FIG._11A

```
                        118                                                    166
        T._reesei       MIWLGKYGDIGPIGSS....QGTVNVGGQSWTLYYGYNGAMQV......YSFVAQT.NTT
  H._schweinitzii       MIWLGKYGDIGPIGSS....QGTVNVGGQTWTLYYGYNGAMQV......YSFVAQS.NTT
  A._aculeatus__*       MIWLARYGGVQPIGSQ....IATATVDGQTWELWYG.....ANGSQKTYSFVAPT.PIT
    A._kawachii_2       MIWLARYGGVQPLGSQ....IATATVEGQTWELWYG.....VNGAQKTYSFVAAN.PIT
        H._grisei       MIWLARYGGIYPIGTF....HSQVNLAGRTWDLWTGYNGNMRV......YSFLPPSGDIR
     G._roseum_Rj_1     MIWLANLGGLTPIGSP....IGTVKAAGRDWELWDGYNGAMRV......YSFVAPS.QLN
     G._roseum_PA_3     MIWLGKLGDIYPIGNS....IGRVEAANREWDFLVGYNGAMKV......FSFVAPS.PVT
     G._roseum_Rj_4     MIWLANLGGLTPIGSP....IGTVKAAGRDWELWDGYNGAMRV......YSFVAPS.QLN

                        167                                                    218
        T._reesei       NYSGDVKNFFNYLRDNKGYNAAGQYV..LSYQFGTEPF..TGSGT.LNVASWTASI.N..
  H._schweinitzii       SYSGDVKNFFNYLRDNKGYNAGGQYV..LSYQFGTEPF..TGSGT.LNVASWTASI.N..
   A._aculeatus         SFQGDVNDFFKYLTQNHGFPASSQYLI..TLQFGTEPF..TGGPATLSVSNWSASVQQAG
    A._kawachii_2       SFQGDINDFFKYLTQNHGFPASSQYLIILALQFGTEPF..TGGPATLNVADWSASVQ...
        H._grisei       DFSCDIKDFFNYLERNHGYPAREQNLIV..YQVGTECF..TGGPARFTCRDFRADL....
     G._roseum_Rj_1     SFDGEIMDFFYVVKDMRGFPADSQHL..LTVQFGTEPI..SGSGAKFSVSHWSAKLG...
     G._roseum_PA_3     LFDGNIMDFFYVMRDMQGYPMDKQYL..LSLQFGTEPF..TGSNANFSCWYFGAKIK...
     G._roseum_Rj_4     SFDGEIMDFFYVVKDMRGFPADSQHL..LTVQFGTEPI..SGSGAKFSVSHWSAKLG...


   A._aculeatus         EPWQNGAGLAVNSFSSTV
     H._grisei          ..W
```

# FIG._11B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4760025 A, Estell
- US 4752585 A, Koths
- US 4732973 A, Barr
- US 5605793 A, Steamer
- WO 9524471 A
- WO 9931255 A
- US 5185258 A
- WO 9623874 A
- US 194664 A
- WO 9418314 A
- US 5264366 A

### Non-patent literature cited in the description

- **Siezen et al.** *Protein Engineering,* 1991, vol. 4 (7), 719-737
- **Gray et al.** *J. Bacteriology,* 1986, vol. 166, 635-643
- **Takkinen et al.** *J. Biol. Chem.,* 1983, vol. 258, 1007-1013
- **Ihara et al.** *J. Biochem.,* 1985, vol. 98, 95-103
- **Nakajima et al.** *Appl. Microbiol. Biotechnol.,* 1986, vol. 23, 355-360
- **Rogers.** *Blochem. Blophys. Res. Commun.,* 1985, vol. 128, 470-476
- **Janecek.** *Eur. J. Biochem.,* 1994, vol. 224, 519-524
- **Feng et al.** *J. Molec. Evol.,* 1987, vol. 35, 351-360
- **Holm.** *Protein Engineering,* 1990, vol. 3 (3), 181-191
- **Buisson et al.** *EMBO Journal,* 1987, vol. 6, 3909-3916
- **Qlan et al.** *Biochemistry,* 1994, vol. 33, 6284-6294
- **Larson et al.** *J. Mol. Biol.,* 1994, vol. 235, 1560-1584
- **Matsuura et al.** *J. Blochem. (Tokyo),* 1984, vol. 95, 697-702
- **Boel et al.** *Biochemistry,* 1990, vol. 29, 6244-6249
- **Vallee et al.** *J. Mol. Biol.,* 1994, vol. 236, 368-371
- **Kadzlola.** *J. Mol. Biol.,* 1994, vol. 239, 104-121
- **Suzuki et al.** *J. Blochem.,* 1990, vol. 108, 379-381
- **Lee et al.** *Arch. Biochem. Blophys,* 1991, vol. 291, 255-257
- **Chang et al.** *J. Mol. Biol.,* 1993, vol. 229, 235-238
- **Mizuno et al.** *J. Mol. Biol.,* 1993, vol. 234, 1282-1283
- **Machlus et al.** *J. Mol. Biol.,* 1995, vol. 246, 545-549
- **MacGregor et al.** *Biochem. J.,* 1989, vol. 259, 145-152
- **Jaspersen.** *J. Prot. Chem.,* 1993, vol. 12, 791-805
- **MacGregor.** *Starke,* 1993, vol. 45, 232-237
- **Janecek.** *FEBS Letters,* 1993, vol. 316, 23-26
- **Janecek et al.** *J. Prot. Chem.,* 1993, vol. 12, 509-514
- **Holm et al.** *Protein Engineering,* 1990, vol. 3, 181-191
- **Vihinen et al.** *J. Biochem.,* 1990, vol. 107, 267-272
- **Dale, J.W.** Molecular Genetics of Bacteria. John Wiley & Sons, 1989
- **Wells et al.** *Nucleic Acid Research,* 1983, vol. 11, 7911-7925
- **Haima et al.** *Mol. Gen. Genet.,* 1987, vol. 209, 335-342
- **Sanger et al.** *Proc. Natl. Acad. Sci. U.S.A.,* 1977, vol. 74, 5463-5487
- **Anagnostopoulos et al.** *J. Bacter.,* 1961, vol. 81, 741-746
- **Stahl et al.** *J. Bacter.,* 1984, vol. 158, 411-418
- **Henner et al.** *J. Bacter.,* 1988, vol. 170, 296-300
- **Hoch.** *J. Bact.,* 1983, vol. 154, 1513-1515
- **Bradford.** *Anal. Biochem.,* 1976, vol. 72, 248